(19) Europäisches Patentamt
European Patent Office
Office européen des brevets

(11) **EP 3 787 488 B1**

(12) **EUROPEAN PATENT SPECIFICATION**

(45) Date of publication and mention
of the grant of the patent:
**25.12.2024 Bulletin 2024/52**

(21) Application number: **19722546.9**

(22) Date of filing: **29.04.2019**

(51) International Patent Classification (IPC):
***A61B 5/021*** *(2006.01)* ***A61B 5/029*** *(2006.01)*

(52) Cooperative Patent Classification (CPC):
**A61B 5/029; A61B 5/02125**

(86) International application number:
**PCT/EP2019/060845**

(87) International publication number:
**WO 2019/211210 (07.11.2019 Gazette 2019/45)**

(54) **METHOD FOR DETERMINING A CARDIAC STROKE VOLUME**

VERFAHREN ZUR BESTIMMUNG EINES HERZSCHLAGVOLUMENS

MÉTHODE DE DÉTERMINATION D'UN VOLUME D'ÉJECTION SYSTOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2018 DE 102018110394**

(43) Date of publication of application:
**10.03.2021 Bulletin 2021/10**

(73) Proprietor: **Philips Medizin Systeme Böblingen GmbH**
**71034 Böblingen (DE)**

(72) Inventors:
• **PFEIFFER, Ulrich**
**5656 AE Eindhoven (NL)**
• **REGH, Stephan**
**5656 AE Eindhoven (NL)**
• **STOLZE, Benjamin**
**5656 AE Eindhoven (NL)**

(74) Representative: **Philips Intellectual Property & Standards**
**High Tech Campus 52**
**5656 AG Eindhoven (NL)**

(56) References cited:
EP-A1- 2 502 555        WO-A2-2017/100188
US-A1- 2013 006 126     US-A1- 2014 073 962
US-A1- 2015 201 873

EP 3 787 488 B1

**Description**

The current invention relates to a method for determining a cardiac stroke volume. Furthermore, the invention relates to an apparatus for determining a cardiac stroke volume and a computer readable medium for performing the method. In particular, the invention relates to a method, apparatus and computer readable medium, for determining a cardiac stroke volume based on an invasive or noninvasive arterial pulse contour analysis, a so called pulse contour stroke volume, PCSV.

Cardiac output, which is defined as the volume of blood a heart pumps over time, is an important parameter for both diagnosis and management of patients especially during surgery, on intensive care units or emergency departments. The cardiac output is typically measured in liters per minute. Cardiac output, CO, depends on the amount of blood a heart pumps with every stroke or heart beat, which is called cardiac stroke volume, and the number of times the heart beats within a given time, which is called heart rate, HR, and which corresponds to the pulse rate, PR.

Conventionally cardiac output or also the stroke volume may be measured using pulmonary artery thermodilution, where a well-defined small amount of cold liquid at a known temperature is injected into the right atrium and the time dependent blood temperature progression is measured after passage of the right ventricle in the pulmonary artery. Given the known volume, specific weight and specific heat capacity of the injected liquid as well as the specific weight and specific heat capacity of the blood, the time dependent blood temperature progression allows for calculation of the cardiac output using the Stewart-Hamilton equation. The (mean) cardiac stroke volume may be calculated by dividing the cardiac output by the heart rate. However, determining stroke volume using the highly invasive pulmonary artery thermodilution is risky, because of threatening complications, time consuming, rather expensive and impractical in the operating room or emergency department.

To make the cardiac output, CO, comparable between individuals it is usually normalized to body surface area and yields cardiac index, CI in $l/min/m^2$.

Conventionally, there are a number of methods to quantify cardiac output, CO. A mostly less invasive and noninvasively applied CO-method is the so-called pulse contour analysis. Thereby certain characteristics of the arterial blood pressure waveform are used to estimate pulse contour stroke volume, PCSV, beat-by-beat. A further approach is to identify the portion of the arterial blood pressure waveform, which corresponds to ejection of blood by the left ventricle, and to estimate PCSV from that. Thereby, algorithms for determining or estimating the PCSV can take into account the pressure pulse wave area in the ejection phase (i.e. systolic pressure area), arterial compliance, impedance, and systemic vascular resistance, amongst other parameters. Since it is based on blood pressure and time data only, the PCSV estimate obtained is not yet related or calibrated as stroke volume in ml blood to an individual patient, e.g. size and condition.

As indicated above for determining a pulse contour stroke volume (PCSV) it is not required to perform any invasive action. Thus, any noninvasive measured arterial pulse contour related data can be used. Even under emergency surgery, the noninvasive pulse contour will be normally determined nevertheless and pressure values and pressure curves taken for determining the blood pressure, in particular time dependent arterial pressure data or noninvasive tissue pressure pulse waveform, are used for determining the cardiac stroke volume.

However, the present invention is also applicable to less invasively measured pulse contours. Even in such case, an improvement with respect to risk, time and costs is achieved compared to pulmonary artery thermodilution for determining the stroke volume.

US 2014/0073962 A1 refers to a photoplethysmogram for determining a stroke volume of a patient including a photoplethysmogram sensor configured to be secured to an anatomical portion of the patient. Further, the photoplethysmogram system includes a monitor operatively connected to the sensor. The monitor receives a photoplethysmograph signal from the sensor and further includes a pulse training module determining a slope transit time of an upslope of a primary peak of the signal. The pulse training module then determines a stroke volume of the patient as a function of the slope transit time.

US 2013/0006126 A1 discloses an apparatus for determining a stroke volume, wherein the apparatus receives an arterial pressure waveform and is arranged to correct the part of the pressure waveform that relates to a heart beat for an influence of an ectopic heart beat of arterial fibrillation on the pressure waveform or of changes in the pressure waveform's baseline.

EP 2 502 555 A1 discloses a method of determining oxygen delivery, wherein the method comprises the steps of non-invasively measuring the arterial blood pressure waveform, determining a cardiac output from a pulse wave analysis of the blood pressure waveform, non-invasively measuring an oxygen saturation and hemoglobin concentration and calculating an oxygen delivery based on the measured quantities.

US 2015/0201873 A1 refers to a method for determining physiological information based on distortion factors and physiological signals. In particular, the method comprises receiving or determining a value indicative of oxygen saturation and calculating the distortion factors based on the value indicative of the oxygen saturation and the physiological signals.

WO 2017/100188 A2 discloses a method for determining a hemodynamic status of a patient, which utilizes systematic perturbations of a venous return or trend observation over time.

WO 2009/014420 A1 relates to a method for determining a beat-to-beat stroke volume using the waveform of arterial pressure data.

However, it has been found that the (mean) cardiac stroke volume determined using only time dependent arterial pressure data - hereinafter pulse contour stroke volume (PCSV) - shows only moderate agreement and correlation with (mean) cardiac stroke volume determined by reference methods - hereinafter SVref -, for example, pulmonary artery and transpulmonary thermodilution or transesophageal echocardiography. Though PCSV can be calibrated with SVref methods initially or repeatedly, this is invasive with thermodilution methods, but always expensive and very time consuming. Hence application of such calibrated PCSV is impractical during surgery, on intensive care units or emergency departments. Even more important, changes of PCSV of existing methods exhibit poor or moderate correlation, agreement, and concordance with large percentage errors if compared to changes of SVref, providing strong evidence that such PCSV methods cannot track changes of SVref. Thus, methods based only on time dependent arterial pressure data of the patient are not reliable, especially in the operating room or emergency department.

Furthermore, any calibration of PCSV by biologically calibrating or setting the pulse contour stroke volume, PCSV, using a patient's gender, age, height, weight, and body surface area, BSA, is not sufficient. The normalization of CO to BSA results in overestimations in the cardiac index, CI, ($l/min/m^2$), in very obese patients especially. Further any conventional biological setting of uncalibrated PCSV and pulse contour cardiac output (PCCO) is complicated by the fact, that the extrapolation of BSA from average body size with normal fat free mass/adipose mass ratio, FFM/AM ratio, to very large, obese size BSA does not take into account the largely reduced FFM/AM ratio in obese individuals with a much lower AM perfusion.

Moreover, the known PCSV methods exhibit a moderate to poor sensitivity on detecting changes of SV if compared to a gold-standard reference method, resulting in poor correlations, clinically inacceptable large limits of agreement, high percentage errors and imperfect concordance rates. Independent of invasive or noninvasive measurement, for determination of PCSV with high accuracy and precision major improvements are necessary.

Accordingly, it is an object of the current invention to provide an improved method for reliably determining a cardiac stroke volume. In particular, it is an object of the invention to provide a method for improving the determination of a cardiac stroke volume based on any pulse contour analysis. Furthermore, it is an object to increase the dynamic sensitivity of PCSV to better track true changes of PCSV, e.g. in comparison to the changes of SVref. It is also an object of the invention to provide methods for improving any PCSV determination which calculates PCSV for every heartbeat and to increase the dynamic sensitivity of PCSV to better track true changes of the PCSV.

Said object has been addressed with the subject-matter of the independent claims. Advantageous embodiments are disclosed in the dependent claims.

Thus, the invention is based on the main idea to more precisely "calibrate" and/or calculate the conventional measured PCSV by using further important parameters. There are two different starting points. One is an uncalibrated PCSV_uncal which has no dimension and needs to be calibrated to the individual. The other starting point is a conventional PCSV_conv, which is already biologically calibrated to the individual with respect to the biological characteristics and is provided in ml, as described above, e.g. by use of the BSA, but is still less precise in particular with respect to the fast changes of the PCSV compared to the reference PCSV measured by risky and time consuming invasive measurements.

In one aspect of the invention any uncalibrated pulse contour stroke volume, PCSV, is improved by considering the blood flow through the metabolically most active very well perfused fat free mass, FFM, and the metabolically very little active and marginally perfused adipose mass, AM. The biological calibration of any uncalibrated PCSV, in the following first PCSV or PCSV_uncal, can be achieved by a separation of blood flow into a portion through the lean body mass, i.e. fat free mass, FFM, and through the adipose body mass, AM. Hence, both blood flow portions have to be calculated separately and added.

Furthermore, as the cardiac output, CO, decreases with age due to increasing stiffness or decreasing compliance of the arterial system and weakening cardiac performance and depends on demographic gender, normally higher in male individuals, also this influence is considered for providing the second PCSV according to the invention.

In another aspect of the invention a fluid responsiveness parameter, FRP, indicative of heart-lung interaction, induced by any respiration form, e.g. spontaneous breathing or fully controlled mechanical ventilation, is used to improve the first PCSV, either the PCSV_uncal or a conventional PCSV_conv (which is derived using medical data obtained from an individual). So different to the first aspect the application of the FRP is possible to both the uncalibrated PCSV_uncal having no dimension and to the conventional PCSV_conv, which has already a dimension, because it was conventionally calibrated to the individual biological characteristics.

Each given heart which is fluid responsive will have a lower stroke volume before volume supply compared to its stroke volume after volume supply. Vice versa, a given heart which is fluid responsive will have a higher stroke volume before blood volume loss compared to its stroke volume after blood volume loss. This behavior is used for improving the PCSV, either as the PCSV_uncal or as PCSV_conv.

Thus, a fluid responsiveness parameter, FRP, is considered in the inventive method for improving the PCSV calculation, to thereby increase the sensitivity of PCSV to track changes of true stroke volume SVref as measured with a reference method.

The consideration of the FRP allows an improvement in accuracy independently of the mode of respiration, be it e.g. controlled mechanical ventilation, any kind of pressure support ventilation, airway pressure release ventilation, or regular spontaneous breathing, or any other mode of respiration or ventilation resulting in heart-lung interaction, preferably with known or measurable phases of regular heart-lung interaction being long enough to determine FRP reliably. Thus, by using the FRP, the individual health status of the individual is considered for increasing the precision of the PCSV.

The fluid responsiveness parameter, FRP, used for the invention can be represented by e.g. pulse pressure variation, PPV, mean left ventricular ejection pressure variation MEPV, stroke volume variation, SVV, systolic pressure variation, SPV, a systolic pressure area variation, SPAV, or photoplethysmographic variability index, PVI, or any other fluid responsiveness parameter with appropriate sensitivity and specificity, or a combination of several ones with e.g. their relative weighted mean.

MEPV is the variation, caused by heart-lung interaction, of the mean left ventricular ejection pressure, MEP.

SPAV is the variation, caused by heart-lung interaction, of the systolic pressure area, Asys, which is the area enclosed by the arterial pressure curve during the systole. This area is defined from the time of the start of the systole to the beginning of the dicrotic notch, which corresponds to the closure of the aortic valve. Its baseline is defined either

- as horizontal line at the level of diastolic arterial blood pressure, DAP, of the preceding pulse, or
- as straight line between the DAP of the preceding pulse and the DAP of the pulse under consideration.

According to the invention if the FRP is a combination of several parameters, these have to be adjusted to comparable ranges by applying equations of structural regression analyses before generating a weighted mean.

The object is solved by a method for determining a stroke volume, SV, of an individual, comprising the steps of: providing a first pulse contour stroke volume, PCSV_uncal, based on one or more characteristics of a measured arterial blood pressure waveform or providing a conventionally derived pulse contour stroke volume, PCSV_conv, determining at least one perfusion parameter, BioCal, descriptive for the perfusion through the fat free mass and the adipose mass of a body of the individual, and/or determining at least one fluid responsiveness parameter function, f(FRP_norm), depending on a fluid responsiveness parameter, FRP_norm, descriptive for a heart-lung interaction of the individual, and adjusting the first pulse contour stroke volume, PCSV_uncal or the conventionally derived pulse contour stroke volume, PCSV_conv, based on at the least one of the perfusion parameter, BioCal, and/or the fluid responsiveness parameter function, f(FRP_norm), to provide a second pulse contour stroke volume, PCSV_imp.

So, the object can be solved by either applying the perfusion parameter, BioCal, or the fluid responsiveness parameter, FRP_norm. Or the object is solved by applying the BioCal parameter and the FRP parameter, FRP_norm.

When deriving the PCSV_conv in a conventional way being already biologically calibrated, the object is also solved by only applying the FRP parameter, FRP_norm.

Preferably, providing a first pulse contour stroke volume, PCSV_uncal, is based on non-invasive measurement or invasive measurement of arterial blood pressure waveform. Here the first pulse contour stroke volume, PCSV_uncal, is a value derived from the arterial blood pressure waveform having no dimension.

In a preferred embodiment, in order to achieve highest accuracy and precision, the method for determining the stroke volume, SV, of an individual, comprising the steps of: providing the first pulse contour stroke volume, PCSV_uncal, based on one or more characteristics of a measured arterial blood pressure waveform, determining at least one perfusion parameter, BioCal, descriptive for the perfusion through the fat free mass and the adipose mass of a body of the individual, and determining at least one fluid responsiveness parameter function, f(FRP _norm), depending on a fluid responsiveness parameter, FRP_norm, descriptive for a heart-lung interaction of the individual, and adjusting the first pulse contour stroke volume, PCSV_uncal, based on the perfusion parameter, BioCal, and based on the fluid responsiveness parameter function, f(FRP_norm), to provide a second pulse contour stroke volume, PCSV_imp.

Preferably, the step of determining at least one fluid responsiveness parameter, FRP_norm, is based on at least one of an arterial pulse pressure variation, PPV, a mean left ventricular ejection pressure variation MEPV, a stroke volume variation, SVV, an arterial systolic pressure variation, SPV, a left ventricular systolic pressure area variation, SPAV, or a photoplethysmographic variability index, PVI, or any other fluid responsiveness parameter with appropriate sensitivity and specificity, or a combination thereof.

Preferably, the fluid responsiveness parameter, FRP_norm, is determined based on a weighted mean value of the pulse pressure variation, PPV, and the systolic pressure variation, SPV or based on MEPV and SPAV. In a most preferred solution, for optimal tracking of true PCSV changes, the fluid responsiveness parameter, FRP_norm, is determined based on a weighted mean value of the FRPs with highest sensitivity and specificity to heart-lung interaction, e.g. the pulse pressure variation, PPV, the mean left ventricular ejection pressure variation, MEPV, and the left ventricular systolic pressure area variation, SPAV.

Preferably, the fluid responsiveness parameter, FRP_norm, is determined by applying a fluid responsiveness normalization function, f_FRP_MV, for mechanical ventilation or the fluid responsiveness parameter, FRP_norm, is determined by applying a fluid responsiveness normalization function, f FRP_SB, for spontaneous breathing, thereby abolishing contaminating changes of parameters like e.g. tidal volume, lung compliance and chest wall compliance to obtain pure standardized normalized fluid responsiveness parameters.

Preferably, the fluid responsiveness parameter, FRP_norm, is determined by considering a compliance of a respiratory system, Crs, of the individual, wherein the compliance of the respiratory system, Crs, comprises lung compliance, Cls, and chest wall compliance, Ccw, since the various components of the respiratory system may have differing influence on the intrathoracic low blood pressure capacitance system, IBCS.

Preferably, for standardization to tidal volume, TV, the fluid responsiveness parameter, FRP_norm is normalized by using a FRP normalization parameter TVnorm, wherein the FRP normalization parameter, TVnorm, is TV normalized to predicted body weight, PBW, or to the body weight, W, but preferably to the fat free body mass, FFM.

Preferably, the fluid responsiveness parameter, FRP_norm, is adjusted to mechanical ventilation, MV by use of predetermined semi-quantitative information about the chest wall compliance, Ccw.

Preferably, the fluid responsiveness parameter, FRP_norm, is normalized for spontaneous breathing, SB, by use of the lung compliance, Cls, and respiration rate, RR as means for replacement of TVnorm, which is not known in SB in most cases.

Preferably, the step of determining a perfusion parameter, BioCal, descriptive for the different perfusion through the fat free mass and/or the adipose mass of a body of the individual comprises at least one of: determining a fat free mass related pulse contour stroke volume calibration factor, PCSV_FFM_Cal, determining an adipose mass related pulse contour stroke volume calibration factor, PCSV_AM_Cal.

Preferably, the method further comprises determining an individual demographic parameter comprising determining the fat fee mass, FFM, determining the adipose mass, AM, a height, H, of the individual, a biological gender of the individual, and an age, y, of the individual, since these parameters have the largest influence on the absolute level of resting SV.

Preferably, the fat free mass related pulse contour stroke volume calibration factor, PCSV_FFM_Cal, is determined by using a perfusion coefficient, B_FFM, relating to the perfusion through the metabolically most active fat free mass. Alternatively or additionally, the adipose mass pulse contour stroke volume calibration factor, PCSV_AM_Cal, is determined by using a perfusion coefficient, B_AM, relating to the perfusion through the metabolically very little active adipose mass.

Preferably, for determining the fat free mass related pulse contour stroke volume calibration factor, PCSV_FFM_Cal, and/or the adipose mass pulse contour stroke volume calibration factor, PCSV_AM_Cal, the fat free mass layer thickness and/or the adipose mass layer thickness are considered, since, in fully anesthetized and muscle-relaxed patients, the perfusion through FFM also depends on the FFM and AM layer thickness and the perfusion through AM also depends on the AM layer thickness.

Preferably, measuring or providing one or more characteristics of the arterial blood pressure waveform comprises to identify a portion of the arterial blood pressure waveform.

The object is also solved by an apparatus for determining a stroke volume, SV, of an individual, comprising: an arterial blood pressure and waveform monitor for providing a first pulse contour stroke volume, PCSV_uncal, or a conventional pulse contour stroke volume, PCSV_conv, based on medical data obtained from an individual connected to the arterial blood pressure and waveform monitor, a parameter unit for providing a perfusion parameter, BioCal, and/or a heart-lung interaction descriptive parameter, FRP_norm, of the individual, a controller for adjusting the first PCSV_uncal or the conventional PCSV_conv based on the perfusion parameter, BioCal, and/or a heart-lung interaction descriptive parameter, FRP_norm, to provide a second pulse contour stroke volume, PCSV_imp, and a medical device receiving the second PCSV_imp and outputting the second PCSV_imp or related data therefrom.

## BRIEF DESCRIPTION OF THE DRAWINGS

The above and other objects, features and advantages of the present invention will be more apparent from the following detailed description taken in conjunction with the accompanying drawings, in which:

Fig. 1 shows a flow chart for a first method for improving a first pulse contour stroke volume, PCSV_uncal, by means of a perfusion parameter, BioCal, and to output a second pulse contour stroke volume, PCSV_imp,

Fig. 1a shows a flow chart for determining the first pulse contour stroke volume, PCSV_uncal,

Fig. 1b shows a part of arterial blood pressure waveform for determining the first pulse contour stroke volume, PCSV_uncal,

Fig. 2 shows a flow chart for determining a perfusion parameter, BioCal, as a part of the first method for improving a first pulse contour stroke volume;

Fig. 3 shows a model for perfusion of adipose mass, AM, and fat free mass, FFM, depending on layer thickness of adipose mass, AM/H, and layer thickness of fat free mass, FFM/H;

Fig 4 shows examples of relations between a perfusion coefficient of fat free mass, B_FFM and layer thickness of the fat free mass, FFM/H for different levels of muscle tone;

Fig 5 shows examples of relations between a reducing function AM_B_FFM_Red and the layer thickness in adipose mass, AM/H for different levels of muscle tone;

Fig 6 shows a semi-quantitative effect on B_FFM and the reducing function, AM_B_FFM_Red, depending on different degrees of muscle tone of an individual;

Fig 7 illustrates an example of a relation between an adipose mass perfusion coefficient, B_AM, and the layer thickness of adipose mass, AM/H;

Fig. 8 shows a flow chart for a second method for improving a pulse contour stroke volume, PCSV, by means of a fluid responsive parameter function f(FRP_norm);

Fig. 9 shows a flow chart for determining the FR parameter, FRP,

Fig. 10 shows a flow chart for determining a fluid responsiveness parameter function, f(FRP_norm), applied in mechanical ventilation, MV, as a part of the second method for improving a first pulse contour stroke volume;

Fig. 11 shows a schematic lung and factors influencing the PCSV;

Fig. 12 illustrates an influence of a compliance of the chest wall, Ccw, to the heart-lung interaction, HLI, during mechanical ventilation, MV;

Fig. 13 illustrates a semi-quantitative direction and degree of adjustment of the FRP normalization function, f_FRP_MV, depending on a level of the chest wall compliance;

Fig. 14 shows a flow chart for determining a fluid responsiveness parameter function, f(FRP_norm), applied in spontaneous breathing, SB, as a part of the second method for improving a first pulse contour stroke volume;

Fig. 15 illustrates the relation of pulse pressure variation, PPV, as representative fluid responsive parameter, FRP, versus respiratory rate, RR, and an approximation function f_FRP(RR) based on measurement pairs from a dataset of measurements taken in volunteers;

Fig. 16 shows an FRP normalization function f FRP_SB for adults derived from the approximation function f_FRP(RR) in Fig.15;

Fig. 17 shows a semi-quantitative direction and degree of adjustment of the FRP normalization function f_FRP_SB depending on a level of the tidal volume, TV;

Fig. 18 shows a schematic illustration of the influence of compliance of the lung system, Cls, to the heart-lung interaction, HLI, during spontaneous breathing, SB;

Fig. 19 shows a semi-quantitative direction and degree of adjustment of the FRP normalization function f FRP_SB depending on the level of the compliance of the lung system, Cls;

Fig. 20 shows examples of the FRP normalization function f_FRP_SB_RR depending on the relation of the compliance of the lungs, Cls, to a default compliance of the lungs, Cls0, and on the tidal volume, TV;

Fig. 21 shows an example of a form of the fluid responsiveness parameter function f(FRP _norm) resulting from a fit function, ln_fit_fct, based on measurement pairs from a dataset of measurements taken in patients;

Fig. 22 shows a regression diagram illustrating results of a method for determining a PCSV (here PCSV = PCSV_conv) based on the Wesseling algorithm for providing the PCSV_uncal by using a conventional biological calibration parameter, BSACal, without applying the fluid responsiveness parameter function, f(FRP_norm), versus the reference SVref;

Fig. 23 shows a regression diagram illustrating results of a method for determining a PCSV (here PCSV = PCSV_conv) based on an alternative algorithm for providing the PCSV _uncal by using a conventional biological calibration parameter, BSACal, without applying the fluid responsiveness parameter function, f(FRP_norm), versus the reference SVref;

Fig. 24 shows a regression diagram illustrating results of the method for determining PCSV (here PCSV = PCSV _conv) changes, ΔPCSV, related to corresponding initial PCSV based on the Wesseling algorithm for providing the PCSV _uncal by using a conventional biological calibration parameter, BSACal, without applying the fluid responsiveness parameter function, f(FRP_norm), versus the SVref changes, ΔSVref, related to corresponding initial SVref;

Fig. 25 shows a regression diagram illustrating results of the method for determining PCSV (here PCSV = PCSV _conv) changes, ΔPCSV, related to corresponding initial PCSVbased on the alternative algorithm for providing the PCSV_uncal by using a biological calibration, BSACal, without applying the fluid responsiveness parameter function, f(FRP_norm), versus the reference SVref changes, ΔSVref, related to corresponding initial SVref;

Fig. 26 shows a regression diagram illustrating results of a method according to the invention for determining PCSV (here PCSV = PCSV_imp) based on the Wesseling algorithm for providing the first PCSV_uncal by using the inventive perfusion parameter, BioCal, without applying the fluid responsiveness parameter function, f(FRP_norm), versus the reference SVref;

Fig. 27 shows a regression diagram illustrating results of the method according to the invention for determining PCSV (here PCSV = PCSV_imp) based on the alternative algorithm for providing the first PCSV_uncal by using the inventive perfusion parameter BioCal, without applying the fluid responsiveness parameter function, f(FRP_norm), versus the reference SVref;

Fig. 28 shows a regression diagram illustrating results of the method according to the invention for determining PCSV (here PCSV = PCSV_imp) changes, ΔPCSV, related to corresponding initial PCSV based on the Wesseling algorithm for providing the PCSV_uncal by using a perfusion parameter, BioCal, without applying the fluid responsiveness parameter function, f(FRP_norm), versus the SVref changes, ΔSVref, related to corresponding initial SVref;

Fig. 29 shows a regression diagram illustrating results of the method according to the invention for determining PCSV (here PCSV = PCSV_imp) changes, ΔPCSV, related to corresponding initial PCSV based on the alternative algorithm for providing the PCSV_uncal by using a perfusion parameter, BioCal, without applying the fluid responsiveness parameter function, f(FRP_norm), versus the SVref changes, ΔSVref, related to corresponding initial SVref;

Fig. 30 shows a regression diagram illustrating results of a method according to the invention for determining PCSV (here PCSV = PCSV_imp) based on the Wesseling algorithm for providing the first PCSV_uncal by using a perfusion parameter, BioCal, and applying the fluid responsiveness parameter function, f(FRP_norm), versus the reference SVref;

Fig. 31 shows a regression diagram illustrating results of the method according to the invention for determining PCSV (here PCSV = PCSV_imp) based on the alternative algorithm for providing the first PCSV_uncal by using the inventive perfusion parameter BioCal, and applying the fluid responsiveness parameter function, f(FRP_norm), versus the reference SVref;

Fig. 32 shows a regression diagram illustrating results of the method according to the invention for determining PCSV (here PCSV = PCSV_imp) changes, ΔPCSV, related to corresponding initial PCSV based on the Wesseling algorithm for providing the PCSV_uncal by using the inventive perfusion parameter, BioCal, and applying the fluid responsiveness parameter function, f(FRP_norm), versus the SVref changes, ΔSVref, related to corresponding initial SVref;

Fig. 33 shows a regression diagram illustrating results of the method according to the invention for determining PCSV (here PCSV = PCSV_imp) changes, ΔPCSV, related to corresponding initial PCSV based on the alternative algorithm for providing the PCSV_uncal by using the inventive perfusion parameter, BioCal, and applying the fluid responsiveness parameter function, f(FRP_norm), versus the SVref changes, ΔSVref, related to corresponding initial SVref;

Fig. 34 shows the relative errors of the second PCSV_imp determined according to the invention and a conventional PCSV, each related to a reference stroke volume SVref;

Fig. 35 shows an apparatus according to the present invention;

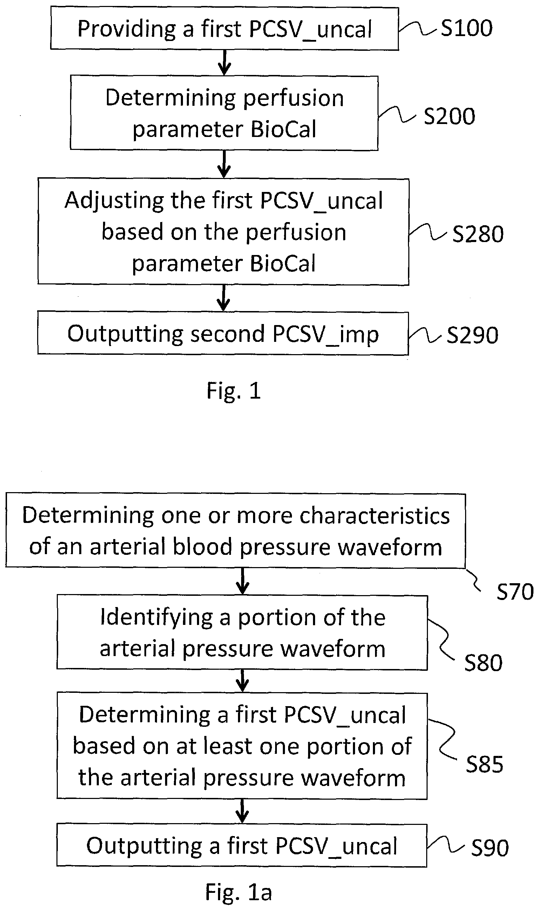

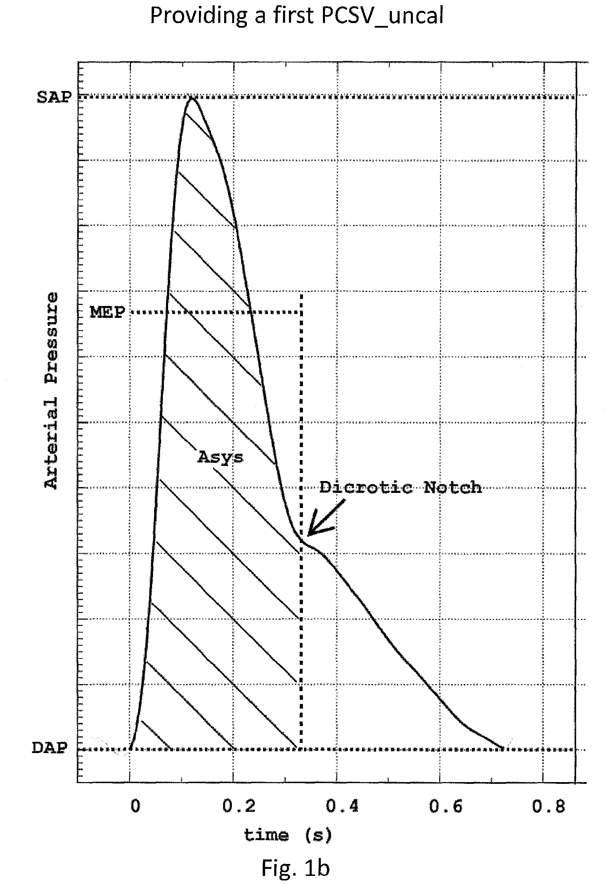

Figure 1 provides a rough overview for the inventive method according to the first embodiment. In the first embodiment a biological calibration based on a perfusion parameter, BioCal, of any uncalibrated pulse contour stroke volume, PCSV_uncal, is made by considering a separation of blood flow into fat free mass, FFM and adipose mass, AM.

According to the flow chart shown in Figure 1 in step S100, the uncalibrated pulse contour stroke volume, PCSV_uncal, -also called first PCSV, is provided. In a next step S200 the perfusion parameter, BioCal, is determined. This will be described in further detail below. After having determined the perfusion parameter, BioCal, this perfusion parameter, BioCal, is applied to the first PCSV_uncal in step S280 to provide the second PCSV_imp in step S290.

Figure 1a provides an exemplary flow chart for providing the first PCSV_uncal. In step S70 a determination or measurement of one or more characteristics of an arterial blood pressure waveform is made. This can be made invasively or noninvasively. So, the result of such determination and/or measurements are parameters of a blood pressure curve of an individual which allow to identify, in step S80, a portion of such blood pressure curve. So, values like amplitude, area enclosed by the curve, et cetera, can be considered to determine the first PCSV_uncal in step S85 and to output the same in step S90. The methods for providing a PCSV_uncal are known in this technological field. An known example for providing such PCSV_uncal is the Wesseling Algorithm (Wesseling algorithm in Chen et al. Comput Cardiol. 2009 Jan 1. The Effect of Signal Quality on Six Cardiac Output Estimator). However, also other algorithm can be used to provide the PCSV_uncal.

An example for providing such PCSV_uncal is illustrated in Figure 1b. Figure 1b illustrates a portion of a blood pressure curve measured, e.g. by use of a high-fidelity oscillometry hydraulic blood pressure cuff or invasively by direct blood pressure measurement using a fluid column and a pressure transducer. In a simple exemplary way the first PCSV_uncal is determined by using blood pressure, pulse pressure, the systolic pressure area, Asys, indicated hatched, and other parameters such as aortic compliance and arterial impedance. The systolic pressure area, Asys, is the area enclosed by the arterial pressure curve during the systole (i.e. ejection phase of the left ventricle), which is defined from the time of the start of the systole, peaking with a systolic arterial blood pressure, SAP, to the beginning of the dicrotic notch, which corresponds to the closure of the aortic valve. Its baseline is defined either

- as horizontal line at the level of diastolic arterial blood pressure, DAP, of the preceding pulse (as in Fig. 1b), or
- as straight line between the DAP of the preceding pulse and the DAP of the pulse under consideration.

The mean left ventricular ejection pressure, MEP, is the mean value of the arterial pressure curve during the systole. The MEP is illustrated in Figure 1b as a horizontal line.

As indicated above, the stroke volume of an individual depends on individual biometric/demographic parameters like body weight, W, comprising of fat free mass, FFM, and adipose mass, AM, body height, H, age and gender.

So the first major determinant of a stroke volume, SV, in an algorithm using an aggregate formula for determining the SV, are the individual biometric/demographic parameters.

The second major determinant of a SV of an individual is the hemodynamic status. So, a pulse contour evaluation of a measured arterial blood pressure pulse waveform results in dynamic input parameters from which an uncalibrated PCSV (PCSV _uncal) is calculated.

The first embodiment according to the invention BioCal provides a calibration function for scaling any given PCSV_uncal to the absolute PCSV value according to the individual biometric/demographic input parameters.

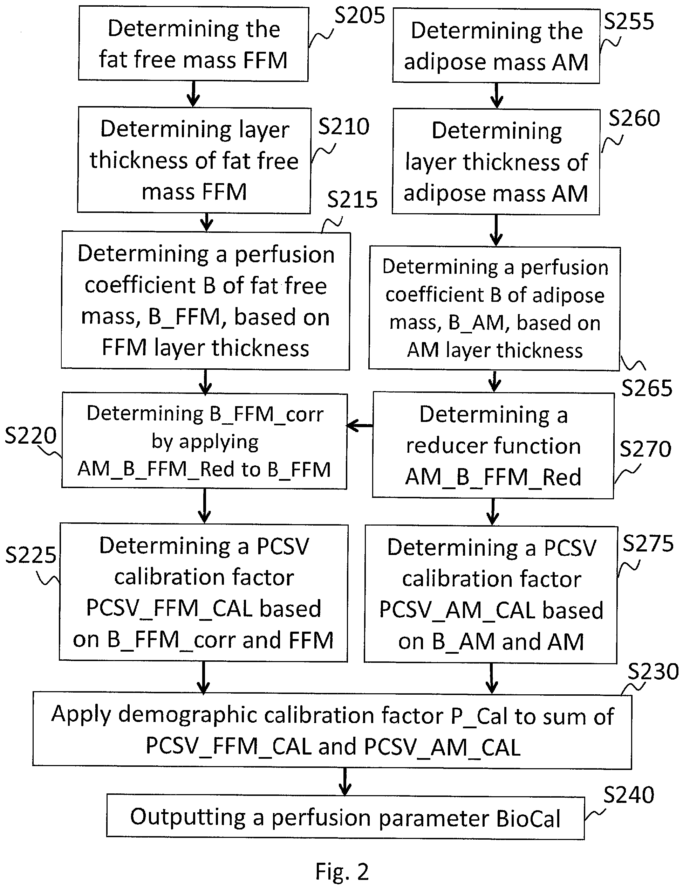

In Figure 2 a detailed flow chart for determining the perfusion parameter, BioCal, is given. As indicated in the first embodiment of the present invention a separation of fat free mass and adipose mass is made. Thus, in step S205 and S255, the fat free mass, FFM and the adipose mass, AM are determined respectively.

The fat free mass, FFM, and adipose mass, AM, determination is explained in detail in the following.

FFM (kg) and AM (kg) represent the two portions of an individual's body weight, W (kg), according to formula 1

$$(1) \qquad W \text{ (kg)} = FFM \text{ (kg)} + AM \text{ (kg)}$$

FFM is the metabolically highly active mass of the body with large oxygen consumption and therefore accounts for the, by far, largest portion of SV. AM is the mass of the metabolically very little active tissue with low oxygen consumption and therefore accounts for a, by far, minor portion of SV.

For estimation of FFM (kg) and AM (kg) the equations of Yu, Heitmann and Janmahasatian in Yu et al. BMC Pharmacol Toxicol. 2013 Oct 14. Lean body mass: the development and validation of prediction equations in healthy adults are used and are averaged to:

FFM (kg) = ( 56.128 kg + (1.3016 + 9270 / (8780 + 244 m$^2$/kg •BMI (kg/m$^2$)))•W (kg) - 2.2268 m$^2$•BMI (kg/m$^2$) - 0.1134 kg/y•age (y) ) / 3, for female individuals --> FFM (kg) = ( 66.068 kg + (1.4138 + 9270 / (6680 + 216 m$^2$/    (2) kg •BMI (kg/m$^2$)))•W (kg) - 2.2268 m$^2$•BMI (kg/m$^2$) - 0.1134 kg/y•age (y) ) / 3, for male individuals

$$(3) \qquad AM \text{ (kg)} = W \text{ (kg)} - FFM \text{ (kg)}$$

Equation (2) is an example for prediction of FFM and can be replaced by further improved prediction equations.

For adolescents, children and infants other appropriate FFM and AM estimation equations are used and are averaged.

It is important to note that such FFM and AM estimations relate to the corresponding condition in a healthy status and in general assume that 1) the water contents of FFM is constant and 2) AM contains no or very little water. Hence, when applied in a clinical condition of a patient, not the actual weight, which may be falsified by e.g. generalized edema or an effusion, may be entered in such equations but only the last known pre-illness weight.

Based on the determination of the FFM and AM a relationship between stroke volume, SV(ml) and the FFM and AM can be given SV(ml) ~ B_FFM (ml/kg) • FFM (kg) + B_AM (ml/kg) • AM (kg) + ... + B_Const (ml),          (4)

where B_FFM is a perfusion coefficient related to FFM perfusion, B_AM is a perfusion coefficient related to AM perfusion and B_Const accounts for other influences on SV. The perfusion coefficients B_FFM and B_AM are determined in Steps S215 and S265 respectively.

In Collis et al. Circulation. 2001 Feb 13;103(6). Relations of stroke volume and cardiac output to body composition: the strong heart study and Corden et al. J Cardiovasc Magn Reson. 2016 May 31; 18(1):32. Relationship between body composition and left ventricular geometry using three dimensional cardiovascular magnetic resonance, it has been found to differentiate B_FFM and B_AM for women and men.

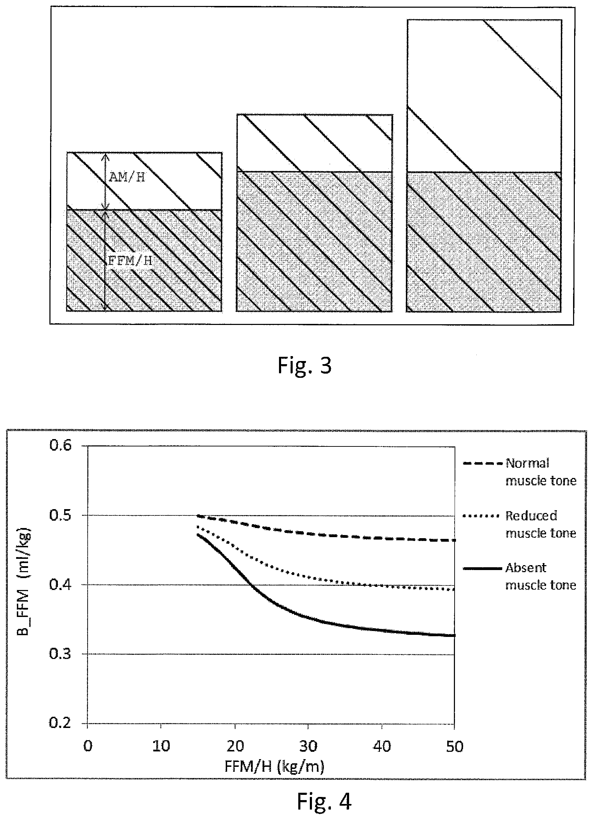

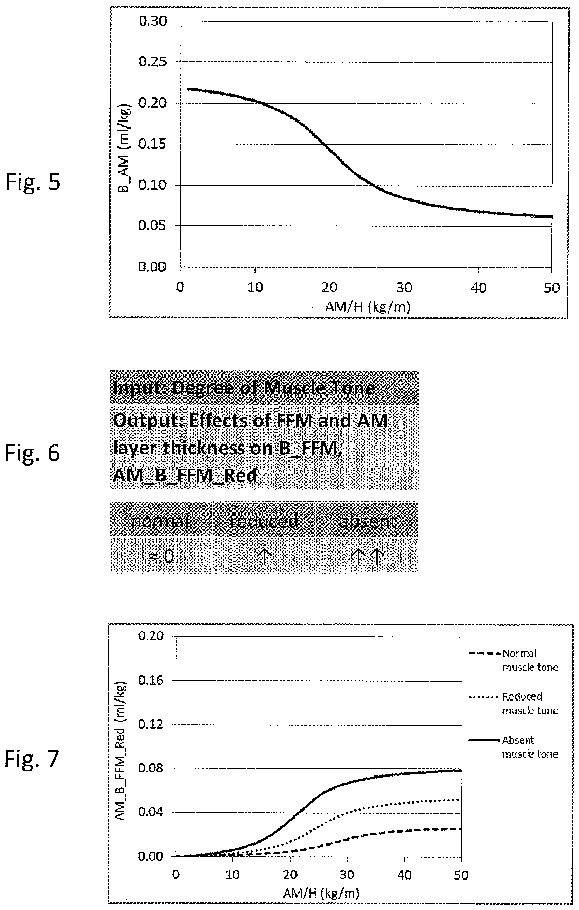

Applying different, but constant perfusion coefficients B_FFM and B_AM considers a difference in the FFM's blood flow per mass FFM_perf (ml/min/0.1 kg) and in AM's blood flow per mass AM_perf (ml/min/0.1 kg) but assumes a perfusion behavior of the FFM and AM portion independent from the relationship of FFM and AM. However, B_FFM and B_AM are not constant (Fig. 4 and Fig. 7).

The blood flow through AM, AM_perf (ml/min/0.1kg), referred to AM (kg) and the perfusion coefficient B_AM strongly depend on the body mass' AM share AM% = AM (kg)/W (kg), indicating that perfusion coefficient B_AM has to be adapted individually. However, the perfusion coefficient B_AM dependency on AM% is not sufficient to describe the variability ofB_AM, because it includes no information of the absolute level of AM.

Thus, according to the invention a simple model is provided that uses AM and FFM distributions over height of an individual to determine the influences on perfusion coefficients B_AM and B_FFM.

In a simplifying model AM and FFM are represented as two corresponding layers as illustrated in Figure 3. The FFM layer thickness is estimated by FFM/H in step S210. The AM layer thickness is estimated by AM/H, in step S260.

Normal values of FFM/H, AM/H and other biometric/demographic parameters are given in the table below.

| Normal values of biometric/demographic parameters | | | | | | | | |
|---|---|---|---|---|---|---|---|---|
| Age (y) | Gender | Height (m) | Weight (kg) | BMI ($kg/m^2$) | FFM (kg) | AM (kg) | FFM/H (kg/m) | AM/H (kg/m) |
| 50 | female | 1.70 | 60 | 21 | 41 | 19 | 24.0 | 11.3 |
| 50 | male | 1.80 | 70 | 22 | 56 | 14 | 31.2 | 7.7 |

The model shown in Figure 3 is based on these considerations and appropriate perfusion functions have been developed to describe the behavior of perfusion coefficients B_FFM and B AM.

In Figure 3 the white portion is the adipose mass, AM, and the gray portion is the fat free mass, FFM. As shown in Figure 3 the AM (white) and FFM (gray) perfusion depends on layer thicknesses AM/H and FFM/H, where H = body height. The density of hatching indicates the level of perfusion - dense hatching = higher perfusion (left side in Figure 3), wide hatching = lower perfusion (right side in Figure 3). As can be easily recognized in case of a thin FFM layer the perfusion through FFM is higher as in case of a thicker FFM layer. But also the perfusion through AM and FFM in case of a thinner AM layer is higher than in case of a thicker AM layer.

In patients with low or without muscular tone, e.g. unconscious or anaesthetized and neuro muscular blockage patients, the muscles lack their structural supporting function. Therefore, the weight of FFM layers influences perfusion coefficient B_FFM gravitationally, because this weight compresses large parts of FFM, i.e. the muscles, inner abdominal organs etc. Thus, the perfusion coefficient B_FFM decreases with increasing layer thickness of FFM/H.

Also it has been recognized that perfusion coefficient B_FFM is reduced with increasing layer thickness AM/H of the adipose mass, AM.

In patients without muscular tone an AM layer thickness reduces the perfusion coefficient B_FFM gravitationally with increasing AM layer, because the increasing subcutaneous fat layer additionally compresses large parts of FFM.

Furthermore, it has been found that the perfusion coefficient B_AM decreases gravitationally with increasing AM/H due to lack of a supporting structure in adipose tissue. Moreover, the perfusion coefficient B_AM is not influenced by the layer thickness of FFM/H.

So, a function B_FFM(FFM/H) as shown in Figure 4 decreases with increasing FFM/H and preferably is a nonlinear function, e.g. an arctan, sigmoid or logarithmic function, preferably described by

$$\text{B\_FFM(FFM/H) (ml/kg)} = \text{a\_FFM (ml/kg)} \cdot \arctan[(\text{FFM/H (kg/m)} - \text{b\_FFM (kg/m)}) \cdot \text{c\_FFM (m/kg)}] + \text{d\_FFM (ml/kg)}, \tag{5}$$

wherein a_FFM, b_FFM, c_FFM and d_FFM are individual FFM related calibration coefficients and constants with B_FFM being in a range of 0.2 ... 0.7 ml/kg.

Exemplary courses of B_FFM(FFM/H) are given in Figure 4 showing relations between B_FFM and FFM/H for different levels of muscle tone showing nonlinearly decreasing B_FFM with increasing FFM/H.

According to the flow chart in Figure 2 the layer thickness of FFM and AM needs to be determined in step S210 and S260, respectively. This is made using the model given in Figure 3. Based on these layer thicknesses FFM/H and AM/H the respective perfusion coefficients B_FFM and B_AM are determined in step S215 and S265, respectively.

In a next step S220 the perfusion coefficient B_FFM is adjusted by applying a reducing function, AM_B_FFM _Red(AM/H). The reducing function may be also replaced by an attenuation function. In the following only the application of a reducing function is described. The reducing function, AM_B_FFM_Red describes the effect of the thickness of AM on the perfusion coefficient B_FFM. The reducing function, AM_B_FFM_Red increases with increasing AM/H and preferably is a nonlinear function, e.g. an arctan, sigmoid or logarithmic function, exemplary shown in Figure 5 for different levels of muscle tone.

The reducing function, AM_B_FFM_Red, increases with increasing AM/H and preferably is a nonlinear function, e.g. an arctan, sigmoid or logarithmic function, described in formula 6,

9

$$AM\_B\_FFM\_Red(AM/H) \text{ (ml/kg)} = a\_att \text{ (ml/kg)} \cdot \arctan[(AM/H \text{ (kg/m)} - b\_att \text{ (kg/m)}) \cdot c\_att \text{ (m/kg)}] + d\_att \text{ (ml/kg)}, \quad (6)$$

where a_att, b_att, c_att and d_att are individual AM related calibration coefficients and constants with AM_B_FFM_Red being in a range of 0 ... 0.2 ml/kg.

Going back to step S220 in Figure 2, a corrected perfusion coefficient, B_FFM_corr, is the difference of the preliminary B_FFM(FFM/H) and AM_B_FFM_Red(AM/H), described in formula 7

$$B\_FFM\_corr \text{ (FFM/H, AM/H) (ml/kg)} = B\_FFM \text{ (FFM/H) (ml/kg)} - AM\_B\_FFM\_Red \text{ (AM/H) (ml/kg)} \quad (7)$$

So, it has been found that depending on the level of consciousness, anesthesia and/or muscle relaxation the perfusion coefficient B_FFM(FFM/H) and the reducing function AM_B_FFM_Red(AM/H) have to be adapted.

Figure 6 illustrates a semi-quantitative effect on B_FFM and on the reducing function, AM_B_FFM_Red, which depends on different degrees of muscle tone of an individual. In the state of absent muscle tone (e.g. fully anaesthetized and muscle-relaxed patients) the effects of FFM and AM layer thickness on B_FFM and AM_B_FFM_Red are most pronounced as shown in Figures 4 and 5. In the state of normal, resting muscle tone the afore mentioned effects are least pronounced, and the state of reduced muscle tone (e.g. unconscious patients) the effects are moderately pronounced.

But, also the function B_AM(AM/H) decreases with increasing AM/H and preferably is a nonlinear function, e.g. an arctan, sigmoid or logarithmic function. It is given by formula 8,

$$B\_AM(AM/H) \text{ (ml/kg)} = a\_AM \text{ (ml/kg)} \cdot \arctan[(AM/H \text{ (kg/m)} - b\_AM \text{ (kg/m)}) \cdot c\_AM \text{ (m/kg)}] + d\_AM \text{ (ml/kg)}. \quad (8)$$

where a_AM, b_AM, c_AM and d_AM are individual AM related calibration coefficients and constants with B_AM being in a range of 0 ... 0.4 ml/kg.

An exemplary course of B_AM(AM/H) is given in Figure 7 showing that the perfusion coefficient B_AM of the adipose mass AM decreases with increasing layer thickness AM/H of the AM.

Alternatively, perfusion coefficient B_AM(AM/H) can be replaced by a function with similar behavior describing the AM perfusion decreasing with increasing AM/H. The perfusion coefficient B_FFM(FFM/H) can be replaced by a function with similar behavior describing the FFM perfusion decreasing with increasing FFM/H and further decreasing with increasing AM/H.

Further alternatively, AM/H is replaced by the ratio of AM and body surface area AM/BSA or by $AM/H^2$ and FFM/H is replaced by the ratio of FFM and body surface area FFM/BSA or by $FFM/H^2$.

If a differentiation of FFM and AM is not possible, because pre-illness weight and/or age is unknown as sometimes occurs with unidentified or unknown emergency patients transferred to intensive care units, then an approximation of this relationship may be achieved with W/H or W/BSA or $W/H^2$ or PBW/H or PBW/BSA or $PBW/H^2$, with PBW being the predicted body weight of an individual.

Going back to Figure 2, steps S225 and S275 an individual static pulse contour stroke volume, PCSV calibration factors related to fat free mass FFM and adipose mass AM are determined by use of the perfusion coefficients B_FFM_corr and B_AM_corr, respectively.

$$(9) \quad PCSV\_FFM\_Cal \text{ (ml)} = B\_FFM\_corr \text{ (ml/kg)} \cdot FFM \text{ (kg)},$$

$$(10) \quad PCSV\_AM\_Cal \text{ (ml)} = B\_AM \text{ (ml/kg)} \cdot AM \text{ (kg)}$$

To obtain the second PCSV_imp of an individual, the first PCSV_uncal is calibrated by using the individual static pulse contour stroke volume, PCSV calibration factors PCSV _FFM _Cal, PCSV_AM_Cal.

$$PCSV \text{ (ml)} = PCSV\_uncal \cdot (PCSV\_FFM \text{ Cal (ml)} + PCSV\_AM\_Cal \text{ (ml)} ) \cdot P\_Cal \quad (11)$$

wherein PCSV_uncal and P_Cal have no dimension.

Furthermore, an additional static, demographic calibration factor P_Cal is applied, as shown in step S230 in Figure 2.

The first PCSV_uncal reflects the actual hemodynamic state of the individual, depending on dynamic influences such as cardiac preload (i.e. dependent on intrathoracic blood volume) and performance (i.e. contractility), aortic compliance, arterial impedance and blood pressure (i.e. cardiac afterload).

P_Cal is a calibration factor that depends on the individual's biometric/demographic characteristics additionally to FFM and AM, that influence the level of SV.

The static, biometric/demographic calibration factor P_Cal can be a sum or product of various biometric/demographic parameters, e.g. as shown in formula 12,

$$(12) \qquad P\_Cal = H\,(m) \cdot coeff\_H\,(1/m) + age\,(yrs) \cdot coeff\_age\,(1/yrs) + const\_gender$$

with coeff_H referring to the individual's body height, coeff_age referring to the individual's age (compliance of the arterial system), const_gender has no dimension and is referring to the individual's biological gender.

In case of transgender a semi-quantitative input information about the individual's physique is needed. This assessment can e.g. be a value within the range 0.0 to 1.0, e.g. 0.7, with male $\hat{=}$ 1.0 and female $\hat{=}$ 0.0

The static, biometric/demographic calibration factor P_Cal can be refined by an additional constant that has no dimension referring to additional parameters influencing the PCSV level that have not been considered by the input data H, age and gender:

$$P\_Cal = H\,(m) \cdot coeff\_H\,(1/m) + age\,(yrs) \cdot coeff\_age\,(1/yrs) + const\_gender + const\_BC \qquad (13)$$

The PCSV calculation can also be further refined by an additional correction constant, const_BC, referring to additional parameters influencing PCSV that have not been considered in the above described PCSV calibration procedure.

So, based on the PCSV_FFM_Cal and PCSV_AM_Cal and demographic calibration factor P_Cal the perfusion parameter BioCal is determined in step S240, which is applied to the first PCSV_uncal.

The second PCSV_imp then adds up to:

PCSV_imp (ml)= PCSV_uncal • [(PCSV _FFM _Cal (ml) + PCSV_AM_Cal (ml) + const_corr (ml)) • P_Cal]
BioCal (ml) = (PCSV_FFM_Cal (ml) + PCSV_AM_Cal (ml) + const-corr (ml)) • P_Cal PCSV_imp (ml) = PCSV     (14)
_uncal • BioCal (ml)

The coefficients and constants of the functions of static input parameters yielding the calibrating constants PCSV_FFM_Cal, PCSV_AM_Cal and P_Calare determined in an evaluation population.

In this evaluation population these coefficients and constants with regard to equation (14) have been obtained by optimizing (manually and by applying the Microsoft Excel solver) the correlation coefficient, slope and intercept of the regression and correlation analyses of PCSV calculated from (14) vs simultaneous gold standard stroke volume SVref measured with transpulmonary thermodilution.

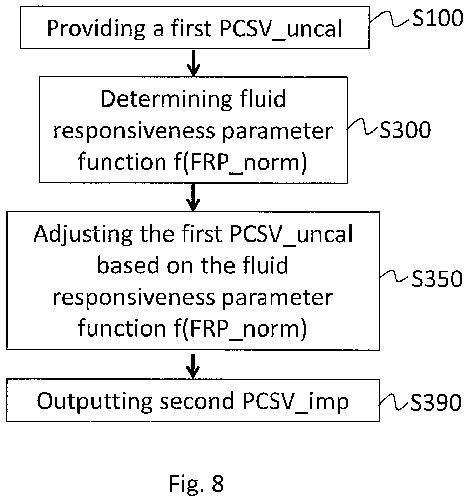

In Figure 8 a flow chart is provided describing a second embodiment of the invention. In the second embodiment of the invention a fluid responsiveness parameter, FRP, is used to better track changes of pulse contour stroke volume. So, a PCSV improvement function is developed that reflects the influence of fluid responsiveness, FR, on stroke volume, which improves the sensitivity of a pulse contour stroke volume algorithm if the heart is responsive to preload volume changes.

Figure 8 illustrates the general procedure for providing the second PCSV_imp in step S390 by using a fluid responsiveness parameter, and in particular a normalized fluid responsiveness parameter FRP_norm and a fluid responsiveness parameter function f(FRP _norm) derived based on the fluid responsiveness parameter FRP_norm. The second PCSV_imp is the PCSV being improved based on the inventive method or apparatus.

In a first step S100, which is similar to step S100 in the first embodiment of the invention described with respect to the Figures 1-7, a first pulse contour stroke volume, PCSV_uncal, is provided. This procedure can either be applied to the first pulse contour stroke volume, PCSV_uncal or to a conventional PCSV result, PCSV_conv, which can be obtained by use of different methods. The PCSV_uncal is a value without any dimension (unitless) based on blood pressure derived data; while the conventional pulse contour stroke volume (PCSV _conv) is measured in ml units and includes into account medical data obtained from an individual based on medical data obtained from said individual.

For providing the first pulse contour stroke volume, PCSV_uncal, it is only required to provide an arterial blood pressure waveform which might be obtained by use of an invasive and/or noninvasive measurement or the first pulse contour stroke volume, PCSV_uncal can be obtained based on a noninvasive tissue pressure pulse waveform and reconstruction of an arterial blood pressure waveform. Based on this arterial blood pressure waveform, the first pulse contour stroke volume, PCSV_uncal, is derived, e.g. by a method as described with respect to Fig. 1b. However, several other methods for providing the first pulse contour stroke volume, PCSV_uncal are possible. For the invention it is not important how the first pulse contour stroke volume, PCSV_uncal is obtained. As explained above the core of the invention is to provide a method to improve the accuracy of the conventional obtained first pulse contour stroke volume, PCSV_uncal.

As described above, a Heart-lung-interaction, HLI, occurs during different situations which need to be differentiated, as the parameters influencing the HLI are different in these situations. HLI occurs during spontaneous breathing, SB, and mechanical ventilation, MV. The MV can be separated into fully controlled mechanical ventilation, CMV, or assisted spontaneous breathing, ASB, and any other ventilation form between SB and CMV.

At first, fluid responsiveness parameters during mechanical ventilation will be discussed. In MV, an external pump (i.e. a mechanical ventilator) presses air or any other ventilation gas mixture into the airways in inspiration and thus inflates the lungs with positive pressure. The inflation of the lungs causes enlargement of the thorax and caudal movement of the diaphragm. Simultaneously, in MV inspiration, the enlarging lungs compress the intrathoracic low blood pressure capacitance system IBCS, which is mainly composed of the vena cava, the right atrium, the right ventricle, the pulmonary circulation, and the left atrium. This compression causes a reduction of venous return, of right ventricular preload and of stroke volume which, after having passed lungs and left heart, again may be detected in the aorta and arterial system as decreased stroke volume or, correspondingly, decreased systolic and pulse pressure.

In order to understand how HLI in MV can be utilized it helps to imagine the lungs to be a testing tool for the relative filling of the IBCS and for estimation of the adequacy of cardiac preload. Relative filling means, that only semi-quantitative graded information is obtained e.g. if cardiovascular filling is adequate, too much, or too little, or much too little. Thereby fluid responsiveness parameters FRPs are usually calculated as percentage variation over at least one respiration of ventilation cycle composed of inspiration and expiration.

Different FRPs, e.g. pulse pressure variation PPV, a mean left ventricular ejection pressure variation MEPV, stroke volume variation SVV, systolic pressure variation SPV systolic pressure area variation SPAV or any other appropriate FRP, can be used alone or combined. In the latter case it is necessary to adjust the different FRPs to comparable levels and ranges, e.g. by applying equations of regression analyses before generating a combined FRP, e.g. calculating a weighted mean.

An example shown for a function to equalize the value range of SPV to fit PPV is:

$$(15) \qquad PPV^* (\%) = coeff \cdot SPV (\%) + const,$$

whereas coeff and const are derived from regression analysis and have no dimension. PPV and PPV* are combined to a FRP as weighted mean:

$$(16) \qquad FRP (\%) = (PPV (\%) + w \cdot PPV^* (\%)) / (1 + w)$$

with w being a weighting factor without dimension.

An example shown for a function to equalize the value range of MEPV to fit PPV is

$$(17) \qquad PPV1^* (\%) = coeff\_MEPV \cdot MEPV (\%) + const\_MEPV$$

with coeff_MEPV, const MEPV being derived from regression analysis and have no dimension.

An example shown for a function to equalize the value range of SPAV to fit PPV is

$$(18) \qquad PPV2^* (\%) = coeff\_SPAV \cdot SPAV (\%) + const\_ SPAV$$

with coeff_SPAV, const_SPAV being derived from regression analysis and have no dimension.

PPV, PPV1* and PPV2* are combined to a FRP as weighted mean:

$$(19) \qquad FRP (\%) = (PPV (\%) + w1 \cdot PPV1^* (\%) + w2 * PPV2^* (\%)) / (1 + w1 + w2)$$

with w1 and w2 being weighting factors without dimension.

The absolute level of FRPs not only depends on the hemodynamic filling status but also on the effectiveness of the FR testing tool, which is the lungs, and the force with which and the conditions under which the test is performed.

Given an unchanged IBCS, e.g. in MV, a smaller tidal volume results in a lower FRP value compared to a larger tidal volume resulting in a higher FRP value. The change of a FRP observable when tidal volume is changed is directly caused by the concomitant change of intrathoracic pressure ITP which reduces transmural pressures in the IBCS and thus lowers its volume.

Thus, normalization of FRP is done, thereby abolishing changes of parameters other than the filling status of the IBCS, like changes in tidal volume.

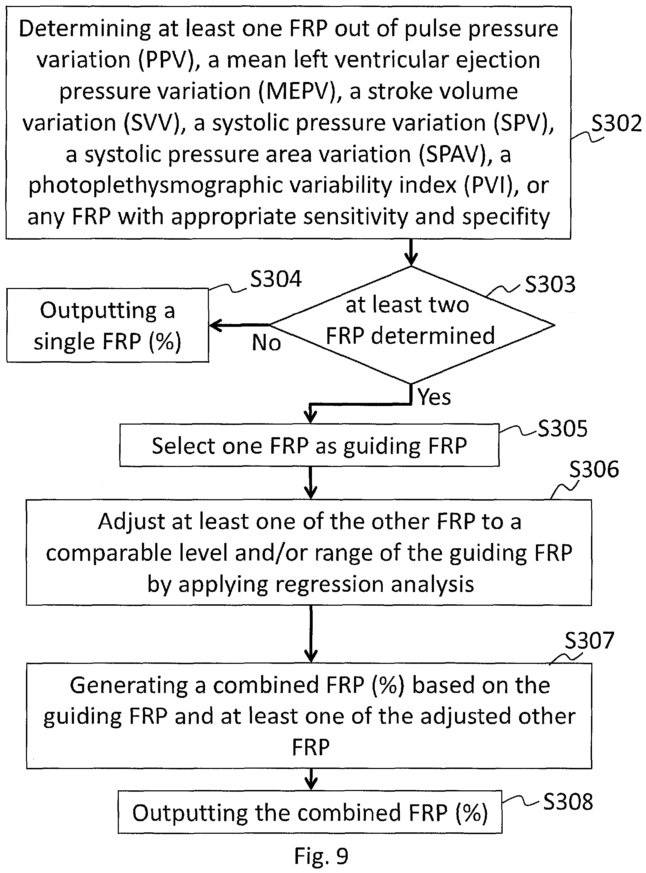

The value of FRP derived in formula (16) or (19) is called combined FRP which is given in %. It is output in figure 9 step S308, which will be explained later in detail. This combined FRP value needs to be normalized yielding a normalized FRP, FRP_norm. Here it has to be differentiated between MV and SB, which will be explained in the following.

Having the normalized fluid responsiveness parameter FRP_norm for MV and SB, the normalized FRP_norm is used generating or determining a fluid responsiveness parameter function f(FRP _norm) in step S300. Thus, at first the fluid responsiveness parameter FRP_norm has to be determined.

In MV, the fluid responsiveness parameter FRP_norm is calculated based on formula 20

$$(20) \qquad FRP\_norm \, (\%) = FRP \, (\%) \bullet f\_FRP\_MV$$

In SB formula 21 is used

$$(21) \qquad FRP\_norm \, (\%) = FRP \, (\%) \bullet f\_FRP\_SB$$

f_FRP_MV and f FRP_SB represent normalization functions which will be explained below.

In formula (20) and (21) the FRP is the combined fluid responsiveness parameter output in step S308 or a single fluid responsiveness parameter output in step S304 in Figure 9. The determination of this FRP will be explained in the following based on Figure 9.

In step S302 at least one FRP is determined. The following blood pressure related data can be used for determining the at least one FRP: pulse pressure variation (PPV), a mean left ventricular ejection pressure variation (MEPV), a stroke volume variation (SVV), a systolic pressure variation (SPV), a systolic pressure area variation (SPAV), a photoplethysmographic variability index (PVI), or any FRP with appropriate sensitivity and specificity.

If only one of the above mentioned FRPs is determined, it is decided in step S303 to output a single FRP in (%) in step S304. If at least two FRPs are determined, one of them will be selected in step S305 as the guiding FRP. As the other of the at least two FRPs have to be adjusted, the adjustment of the other FRP is made in step S306. Thus, the at least one other FRP is adjusted to a comparable level and/or range of the guiding FRP. The adjustment is made by using regression analysis, where coefficients and constants are used to adjust the levels of the different FRPs to the guiding FRP as indicated in formula 15, 17 and 18.

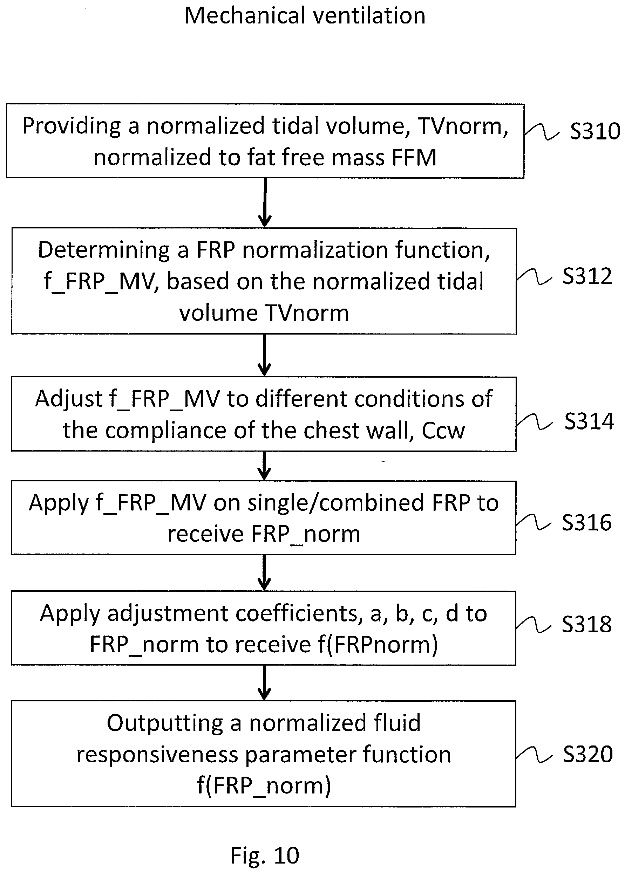

After having adjusted the at least one other FRP to the guiding FRP in step S306, the combined FRP is generated in step S307. The dimension of the combined FRP is %. The single or combined FRP is used for determining the FRP_norm in step S300 in Figure 8. As indicated above the combined or single FRP is normalized by a FRP normalization function in step S316 in figure 10.

In the following, the normalization of the single or combined FRP is described for mechanical ventilation. It is referred to formula 17 mentioned above. So, for normalizing the FRP to derive a FRP_norm the normalization function f_FRP_MV will be deployed.

At first the need for normalization is explained. When considering the lungs as testing tool for the filling status of the IBCS, a standardization is necessary, since the various components of the respiratory system may have differing influence on the IBCS. So, the whole respiratory system and its components needs to be considered, each of which may change remarkably from health to disease of the individual.

One important factor is the compliance, Crs, of the respiratory system. The static compliance of whole respiratory system, Crs, comprises lung compliance per se, Cls, and chest wall compliance, Ccw.

According to Grinnan et al 2005 [2] the following relationship is applicable, given in formula (22)

$$Crs \, (ml/cmH_2O) = Ccw \, (ml/cmH_2O) \bullet Cls \, (ml/cmH_2O) \, /(Ccw \, (ml/cmH_2O) + Cls \, (ml/cmH_2O)) \qquad (22)$$

Crs of normal, adult lungs is 70-100 ml/cmH$_2$O, may be decreased to even 25 ml/cmH$_2$O in acute, severe ARDS (adult respiratory distress syndrome) and chronic lung fibrosis.

The Ccw describes the compliance of the chest wall, i.e. ribcage and diaphragm regarded as one entity. Ccw may be decreased e.g. in sepsis of abdominal origin due to increased intraabdominal pressure and thereby stiffened diaphragm; the same happens with any disease stiffening the rib cage, e.g. in Bechterew's disease. Vice versa Ccw may be increased due to an open abdomen during surgery. Reduced Ccw requires a higher mechanical inspiratory pressure to achieve the same inflation level of the lungs and thus increase ITP to a higher level as compared to a chest wall with normal Ccw. Vice versa this applies to a chest wall with Ccw increase as well.

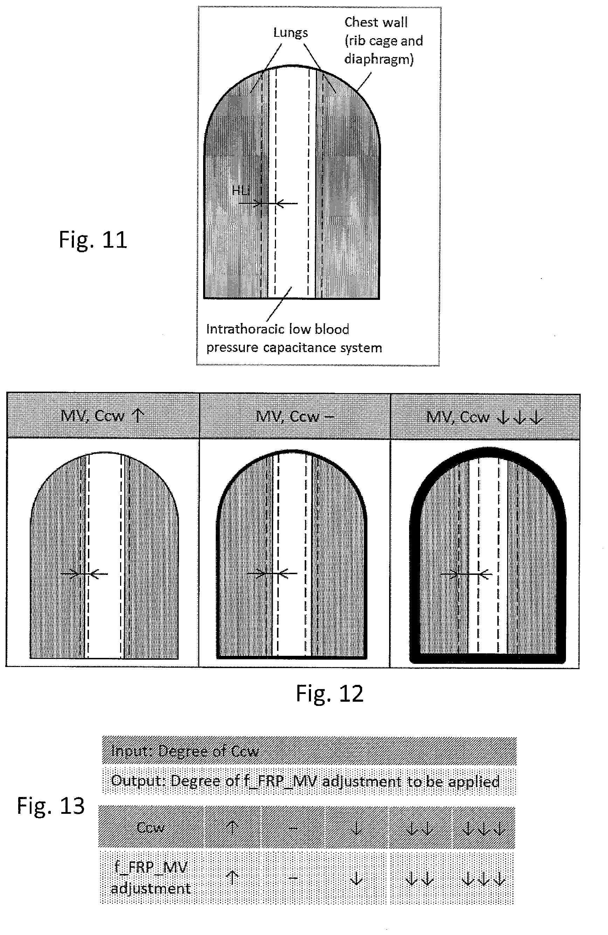

For illustration purpose it is referred to Figures 11 and 12 illustrating the respiratory system including the lungs and the chest wall, both influencing the IBCS.

In usual clinical practice in the operating room a mechanical ventilator is set to CMV with a target tidal volume, peak inspiratory pressure limit, positive end-expiratory pressure and ventilation rate for a given situation. Usually it is impractical or even impossible to perform a standardized test procedure e.g. with a standardized tidal volume. Hence, the resulting FRP values need to be normalized by compensating/removing the parameter(s) influencing FRP apart from the level of the IBCS in order to obtain an inter-individually comparable, normalized FRP_norm.

In mechanically ventilated individuals the changes of FRPs are induced by intrathoracic pressure changes, ITP changes, which are caused by the respective tidal volume TV, which is applied by the ventilator. With IBCS remaining constant a larger TV results in larger FRP values, whereas a smaller TV results in smaller FRP values.

Using the same TV in a patient with a stiffer ribcage with lower Ccw compared to an otherwise completely identical patient with normal ribcage with normal Ccw will result in a higher change of ITP and a higher value of FRPs in consequence.

So the FRP will be normalized based on the tidal volume TV, which has to be normalized as well to make it inter-individually comparable.

In usual clinical practice TV is normalized to predicted body weight PBW as defined by the NIH-NHLBI ARDS Network: PBW calculates according to the formulae (23) and (24),

$$(23) \qquad \text{male, PBW (kg)} = 50 \text{ kg} + 2.3 \text{ kg/in (height (in)} - 60 \text{ in);}$$

$$(24) \qquad \text{female, PBW (kg)} = 45.5 \text{ kg} + 2.3 \text{ kg/in (height (in)} - 60 \text{ in)}$$

This normalization takes into account only gender and height, limited to input between 48 and 84 inches (123 - 213 cm), but not weight and age and therefore is quite rough. The normalization of TV to PBW only based on formulae (23) and (24) does not provide an algorithm for estimation of PBW in infants and children. Furthermore, it assumes a linear relationship between height and PBW, fairly untypical for most biometrical relationships.

So, it is proposed to relate TV to the metabolically most active compartment of the body which is known as lean or fat free body mass FFM. FFM correlates best with oxygen uptake, cardiac output, left ventricular mass, and with variables of lung function, e.g. total lung capacity TLC, wherein the TLC is closely related to maximal lung volume.

The predictive estimation of FFM in kg is explained above in the first embodiment in formulae (2) and (3) and will be applied also here. The estimation of FFM, using other appropriate algorithms, may also be applied to children and infants.

Summarizing, the FRP normalization function used for normalizing the single or combined FRP is calculated based on

$$(25) \qquad \text{f\_FRP\_MV} = \text{TVnorm0 (ml/kg) / TVnorm (ml/kg)}$$

So, the FRP normalization function is normalized with the relation of TVnorm to a default value TVnorm0 of e.g. 8ml/kg FFM in adults, whereas TVnorm is calculated based on TV and FFM based on formula

$$(26) \qquad \text{TVnorm (ml/kg)} = \text{TV (ml) / FFM (kg)}$$

The normalized tidal volume TVnorm is provided in step S310 in figure 10. TV is read from the ventilator used for mechanical ventilation. FRP_norm is equal to FRP if TVnorm = TVnorm0. The FRP normalization function is determined in step S312 based on the normalized TVnorm.

Refinement of the normalization of FRP normalization function with additional coefficient coeff_MV and constant const_MV is possible as given in formula 25

$$(27) \qquad \text{f\_FRP\_MV} = \text{TVnorm0 (ml/kg) / TVnorm (ml/kg)} \cdot \text{coeff\_MV} + \text{const\_MV}$$

e.g. with TVnorm0 = 6 .. 12 ml/kgFFM, coeff_MV = 0.5 .. 2 and const_MV = -1 .. 1 in adults, when PPV and SPV are used as the combined FRP.

The normalization of FRP to TVnorm already compensates for different conditions of Cls, because the influence of MV with constant TVnorm to ITP is the same for any Cls.

As explained above the respiration system further includes the chest wall. Thus, an adaption of f_FRP_MV to the different conditions of the chest wall compliance Ccw is required as made in step S314. Here input data are required that give information if Ccw is normal, increased or decreased and by which degree Ccw is increased or decreased. Preferred graduation of the degrees is:

(a) moderate increase (↑)

(b) normal (-)
(c) moderate decrease ($\downarrow$)
(d) large decrease ($\downarrow\downarrow$)
(e) very large decrease ($\downarrow\downarrow\downarrow$)
but not limited to these.

An increase of Ccw may be observed in e.g. a situation where the abdomen has been opened (moderate increase) or when the chest has been opened (larger to large increase). However, the latter condition is difficult to graduate since it varies largely if the chest is opened partially or completely with being largest if both lungs are freely exposed to atmospheric pressure. Hence the graduation of such situation is not taken into regard because of practical issues.

It is further referred to Figure 12 illustrating the influence of Ccw to HLI in MV, wherein large/low HLI is displayed as large/small distance between vertical dotted lines.

So, the FRP normalization function f_FRP_MV is adjusted depending on direction and level of Ccw. Increased Ccw requires f_FRP_MV to be adjusted to a higher level and vice versa as illustrated schematically in figure 13. The level of adjustment can be made in quantitative steps and will be input by the physician or medical staff based on the health condition and physical characteristics of the chest wall of the individual.

All above described standardization methods that compensate the FRPs for different TVnorm and Ccw generally work at any mode of positive pressure mechanical ventilation.

After having considered the different conditions of the compliance of the chest wall Ccw in step S314 by adjusting the FRP normalization function f_FRP_MV correspondingly. The FRP normalization function f_FRP_MV is applied on the single or combined FRP in step S316 to achieve the normalized FRP_norm.

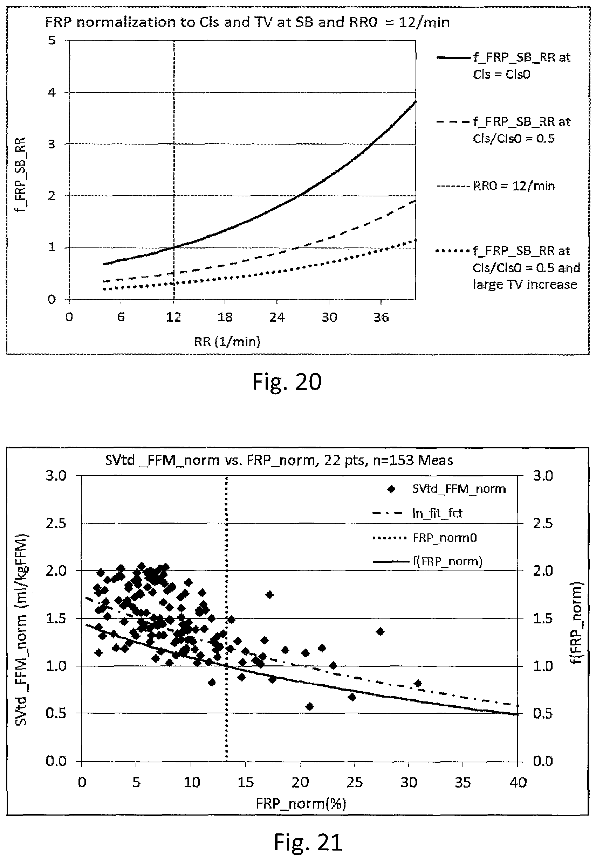

The normalized FRP_norm is used to develop the fluid responsiveness parameter function f(FRP _norm) in step S318 which is a linear or nonlinear function, or a combination of these, that increases the PCSV value at small FRP_norm values and decreases the PCSV value at large FRP_norm values. An exemplary f(FRP_norm) is illustrated in Figure 21.

A possible linear form of the fluid responsiveness parameter function f(FRP _norm) is:

$$(28) \qquad f(FRP\_norm) = a \bullet FRP\_norm\ (\%) + d$$

with a and d having no dimension and a < 0.

A possible basic nonlinear form of the fluid responsiveness parameter function f(FRP _norm) is:

$$(29) \qquad f(FRP\_norm) = a \bullet \ln(FRP\_norm\ (\%)) + d$$

with a and d having no dimension and a < 0
that can preferably be refined by additional adjustment coefficients to:

$$(30) \qquad f(FRP\_norm) = a \bullet \ln(FRP\_norm\ (\%) \bullet b - c) + d$$

with a < 0, wherein the dimensionless adjustment coefficients a, b, c and d are derived based on clinical studies.

The final fluid responsiveness parameter function f(FRP _norm) is output in step S320 for being used in step S350 in figure 8 for improving the first PCSV_uncal based on the fluid responsiveness parameter function f(FRP_norm). In step S390 the second PCSV_imp is output, e.g. on a display of a medical device 330 or is used for controlling an automatic feeder or infusion pump connected or included in the medical device 330.

The underlying formula 29 for calculating the second PCSV_imp is

$$(31) \qquad PCSV\_imp\ (ml) = PCSV\ (ml) \bullet f(FRP\_norm)$$

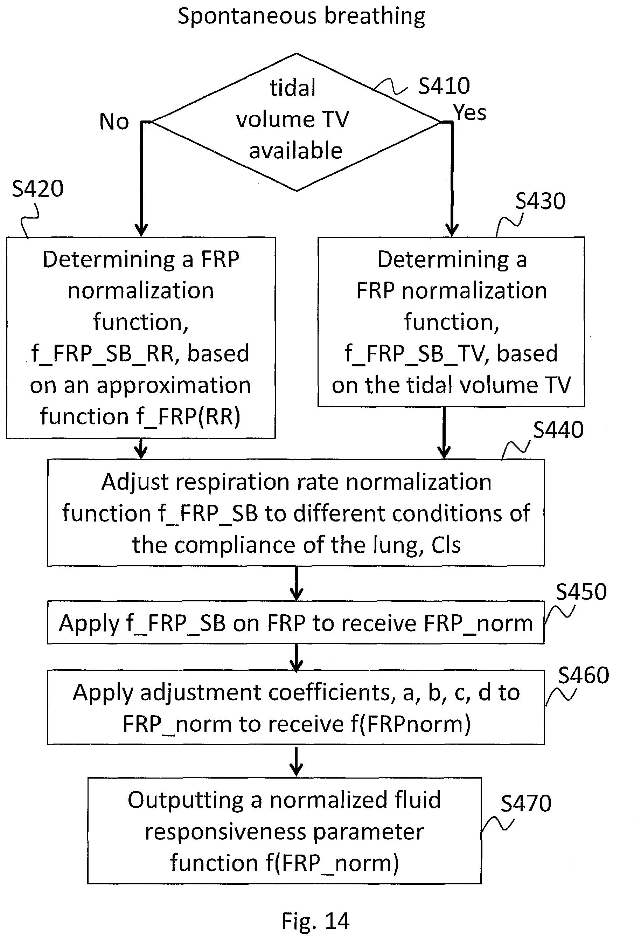

In the following the HLI mechanisms in spontaneous breathing SB and the normalization of fluid responsiveness parameters in spontaneous breathing will be explained with reference to Figures 14-20.

In spontaneous breathing inspiration happens because the intercostal respiratory muscles and the diaphragm contract and increase the volume of the thorax which induces a negative intrathoracic pressure ITP and a negative pressure within the lungs. By this air or any other ventilation gas mixture streams into the lungs, because the atmospheric pressure surrounding the individual is higher than the pressure within the airways of the lungs. Simultaneously, because of the inspiratory negative ITP, venous blood flow from extrathoracic compartments to the IBCS (e.g. vena cava, right atrium) increases, thus right ventricular preload increases and, hence, right ventricular stroke volume.

The increased right ventricular stroke volume travels, beat-by-beat, through the lungs, increases left ventricular end-diastolic volume and stroke volume. The increased left ventricular stroke volume may be detected in the aorta and arterial system by a concomitant rise in e.g. systolic and pulse pressure. In normal expiration this HLI mechanism is reversed, respiratory muscles and the diaphragm relax, the volume of the thorax decreases passively in normal breathing mainly due to elastic recoil upon relaxation of inspiration muscles. ITP and subsequently distal airway pressure is getting positive which causes exhalation towards the lower atmospheric pressure. In forced expiration other internal intercostal and abdominal muscles contract and create a substantially higher positive ITP.

Both, normal and forced expiration cause an increase of ITP, a compression of the IBCS, cause a reduction of venous return, right ventricular preload and stroke volume which again may be detected in the aorta and arterial system by a decrease in e.g. systolic and pulse pressure.

Since all these conditions influence the fluid responsiveness parameters, normalization is needed.

Similar to the situation during mechanical ventilation, the lungs cannot be standardized as testing tool per se without taking the different static lung compliance Cls in healthy and diseased lungs into account.

A stiff lung with reduced Cls (fibrosis etc.) will require a more negative ITP to achieve the same inflation level as compared to a lung with normal Cls. A more negative ITP during spontaneous inspiration causes a higher venous return, higher stroke volume and finally a larger FRP value.

Different from the conditions in MV the influence of Ccw to ITP and thereby to FRP can be considered negligible = not applicable in SB.

Usually neither ITP changes nor tidal volume can be measured in SB without causing stress to the patient or individual. However, tidal volume induced FRP changes need to be normalized as well and static lung compliance taken into account. At the same level of physical activity and environmental condition alveolar ventilation remains constant over time. Any increase in RR results in a lower tidal volume and a respiration rate RR decrease results in a higher tidal volume. In this case respiratory rate RR or a corresponding variable is used for FRP normalization, because it is related to TV and thereby the FRPs. The RR of a spontaneously breathing adult individual may vary in the range of 6 .. 40/min under normal and most pathologic conditions. With constant IBCS a larger RR results in lower TV and thus smaller FRP values, whereas a lower RR results in larger TV and thus larger FRP values.

During SB the normalization function f_FRP_SB for normalizing the single or combined FRPs is any linear or non-linear function that eliminates the RR dependency and Cls dependency of FRP. Any measured FRP value, examples thereof are given above, of an individual at a given RR and lung compliance Cls is normalized to RR and Cls by application of a normalization function f_FRP_SB.

So, depending on whether the tidal volume TV is available (which will rarely be the case), different ways could be used for determining the FRP normalization function, f _FRP_SB, which is applied on the single or combined FRP to achieve the normalized FRP_norm as shown in formula 18 above. With reference to figure 14 in step S410 it is checked whether the tidal volume TV is available. If the tidal volume TV is not available, the FRP normalization function, f FRP_SB is determined based on an approximation function f_FRP(RR) in step S420. If TV is available the FRP normalization function, f_FRP_SB is determined based on the tidal volume in step S430.

At first the determination of the FRP normalization function, f_FRP_SB_RR based on the RR will be described in detail.

The basic form of the FRP normalization function f_FRP_SB_RR can be described as:

$$(32) \qquad f\_FRP\_SB\_RR = RR\ (1/min)\ /\ RR0\ (1/min)$$

f _FRP_SB_RR must increase the FRP values if RR is larger than a default value RR0 of e.g. 12/min and decrease the FRPs if RR is smaller than RR0.

The RR dependency of f_FRP_SB_RR can be obtained from statistical analyses of FRP measurements with simultaneous RR measurements of healthy, euvolemic individuals.

F_FRP _SB_RR can be of the form:

$$(33) \qquad f\_FRP\_SB\_RR = f\_FRP(RR0)\ /\ f\_FRP(RR)$$

with f_FRP(RR) being a respiratory rate correction function, preferably an exponential function, preferably of the following form:

$$(34) \qquad f\_FRP(RR) = a \cdot exp(-b\ (min) \cdot RR\ (1/min))$$

e.g. for adults with a = 5 ... 20%, b = 0.02 ... 0.10 min.

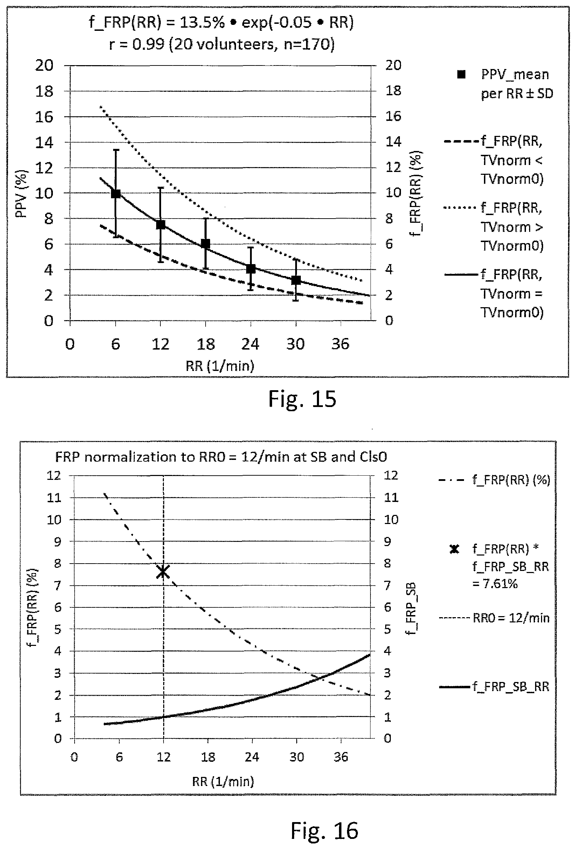

An example for the respiratory rate correction function f_FRP(RR) is provided in Figure 15 illustrating that with increasing RR the PPV is decreasing.

Figure 15 shows results of investigation in 20 adults, healthy, euvolemic (= normovolemic) volunteers who were asked to breathe between 6 and 30 times per minute according to the beat of a metronome (in total n = 170 measurements). Noninvasive PPV was measured as FRP. The squares represent means ($\pm$ standard deviation) of PPV. The shown curve is the exponential approximation function f_FRP(RR) based on these data points.

Further, figure 15 shows that compared to a default value TVnorm0 the function f_FRP(RR) is increased with increased TVnorm and decreased with decreased TVnorm.

Figure 16 shows an example of an FRP normalization function f_FRP_SB_RR (solid line) for adults derived from f_FRP(RR) in figure 15 (dot dashed line in Figure 16) at default lung compliance Cls0, using formulae (33) and (34).

The FRP normalization function f_FRP_SB_RR eliminates the influence of RR to FRP by clamping FRP to the value at RR0 = 12/min, here FRP_norm = 7.61%. The value of f_FRP_SB_RR at RR0 is 1, so the FRP value at RR = RR0 remains unchanged. By multiplication with f_FRP_SB_RR a measured FRP at a RR value smaller than RR0 is decreased and a measured FRP at a RR value larger than RR0 is increased.

It is noticed, that several pathologic conditions cause an increase or a decrease of TV. A TV increase causes an increase of breathing induced ITP changes. Subsequently the FRPs increase at unchanged blood volume of the patient. This pathologically entailed FRP increase has to be corrected.

For further improving the FRP correction, input data is needed that give an estimation if and to which level TV is decreased or increased.

Preferred quantified levels of TV, which needs to be inputted by the physician or medical staff are:

(a) moderate decrease ($\downarrow$)
(b) normal (-)
(c) moderate increase ($\uparrow$)
(d) large increase ($\uparrow\uparrow$)
(e) very large increase ($\uparrow\uparrow\uparrow$)
but not limited to these.

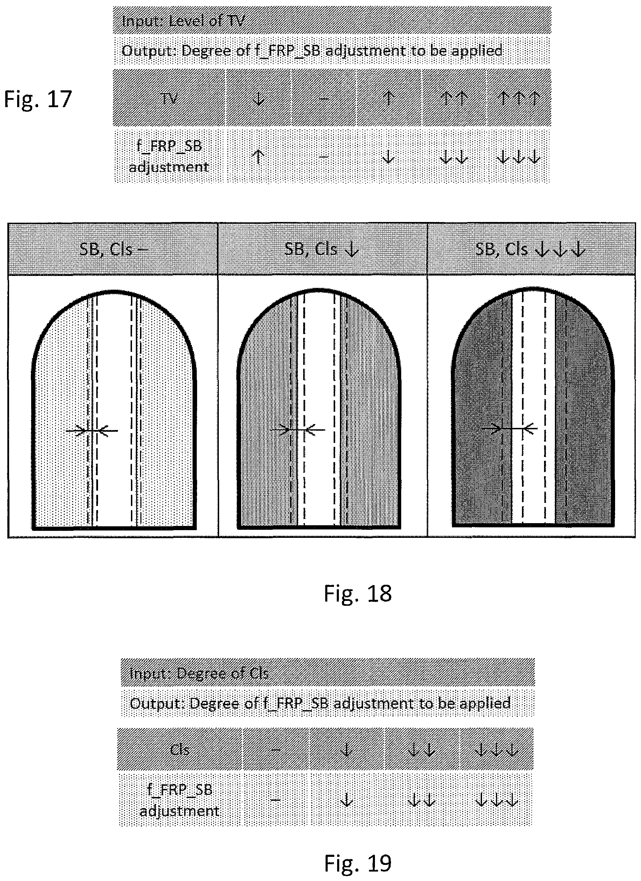

Based on the inputted values the FRP normalization function f_FRP_SB_RR is adjusted depending on direction and level of TV. Increased TV requires f_FRP_SB_RR to be adjusted to a lower level, whereas a decreased tidal volume requires to adjust the f_FRP_SB_RR to a higher level. For illustration of these levels it is referred to figure 17.

Now the determination in step S430 of the FRP normalization function is explained if the tidal volume TV is available.

If TV is available FRPs can be normalized with the TVnorm being TV normalized to FFM in a similar way as in MV by application of a function f_FRP_SB_TV

$$(35) \qquad FRP\_norm\ (\%) = FRP\ (\%) \bullet f\_FRP\_SB\_TV$$

FRP can be normalized with the relation of TVnorm to a default value TVnorm0 of e.g. 8ml/kg FFM for adults.

$$(36) \qquad f\_FRP\_SB\_TV = TVnorm0\ (ml/kg)\ /\ TVnorm\ (ml/kg)$$

Preferably, refinement of the normalization algorithm with additional dimensionless coefficient coeff_MV and dimensionless constant const_MV is possible.

$$f\_FRP\_SB\_TV = TVnorm0\ (ml/kg)\ /\ TVnorm\ (ml/kg) \bullet coeff\_SB\_TV + const\_SB\_TV \qquad (37)$$

Furthermore, an adaption of a FRP normalization function f_FRP_SB, that can be either dependent of RR (f_FRP_SB_RR) or dependent of TVnorm (f_FRP_SB_TV), to the different conditions of static lung compliance Cls further requires input data that gives information if Cls is normal or if and by which degree Cls is increased. This is made in step S440.

Preferred graduation of the degrees of the input data, which is input by the physician or medical staff is:

(a) normal (-)
(b) moderate decrease ($\downarrow$)
(c) large decrease ($\downarrow\downarrow$)
(d) very large decrease ($\downarrow\downarrow\downarrow$) but not limited to these.

Further it is referred to figures 18 and 19 illustrating the different situations of the influence of the Cls on the HLI during SB, wherein a lower Cls is displayed as darker lungs and large/low HLI displayed as large/small distance between vertical dotted lines. So, the left hand illustrated lungs have a normal Cls, whereas the right hand illustrated lungs have a strongly reduced Cls.

The FRP normalization function f_FRP_SB needs to be adjusted depending on direction and level of Cls. So, a decreased Cls requires f FRP_SB to be adjusted to a lower level. A strongly reduced Cls requires to strongly lower the f _FRP_SB.

It is further referred to figure 20 showing an example of FRP normalization functions f_FRP_SB_RR at Cls = Cls0 and Cls/Cls0 = 0.5, using formulae (33) and (34). The value of f FRP_SB_RR at RR0 is equal to the relation Cls/Cls0.

After having adjusted the FRP normalization function f _FRP_SB, the adjusted f FRP_SB is applied to the single or combined FRP in step S450. Here the normalized FRP_norm for SB is achieved.

Similar to MV the adjustment coefficients a, b, c, and d are applied to FRP_norm in step S460 to finally receive the FRP function f(FRP_norm). This FRP function f(FRP_norm) is output in step S470 for being applied to the PCSV_uncal to finally get the PCSV_imp as defined in formula 29, which is provided again here:

$$(29) \qquad \text{PCSV\_imp (ml)} = \text{PCSV (ml)} \cdot \text{f(FRP\_norm)}$$

Summarizing the second embodiment, the stroke volume of an individual's heart which is fluid responsive depends on the hemodynamic filling state. A lower filling state indicated by a larger FRP will result to a lower stroke volume and vice versa. Therefore, the PCSV calculation is improved by taking into account the actual FRP value. To compensate/remove the parameter(s) influencing FRP apart from the level of the IBCS to this PCSV improvement the invention proposes to use a normalized FRP_norm being normalized with TVnorm and Ccw in MV or with RR, TVnorm and Cls in SB.

In the following figures 21 to 34 data of noninvasive high-resolution oscillometry measurements and data of simultaneous invasive measurements in patients during high-risk surgery are shown.

With reference to figure 21 an example of a possible form of f(FRP_norm) is shown that results from a logarithmic fit function, ln_fit_fct, on the dependency of the SVtd_FFM_norm (ml/kgFFM) from FRP_norm, where SVtd_FFM_norm is the FFM part of SVtd normalized to FFM.

The logarithmic fit function ln_fit_fct is based on statistical analysis of data of 22 adult patients with FRP_norm changes > 5%.

f(FRP_norm) is derived from ln_fit_fct by normalization to the value of ln_fit_fct at a default value of FRP_norm (here 1.2 ml/kgFFM at FRP_norm0 = 13.2%).

Furthermore, any multiplication of f(FRP_norm) with a given PCSV increases PCSV at FRP_norm < FRP norm0 and decreases PCSV at FRP_norm > FRP norm0. So, it is an amplification or attenuation function based on normalized FRP values.

Nevertheless, the inventive method provides a high precision of the derived values for PCSV resulting a more reliable diagnosis and better and maybe life-saving reaction of the physician or medical staff during surgery.

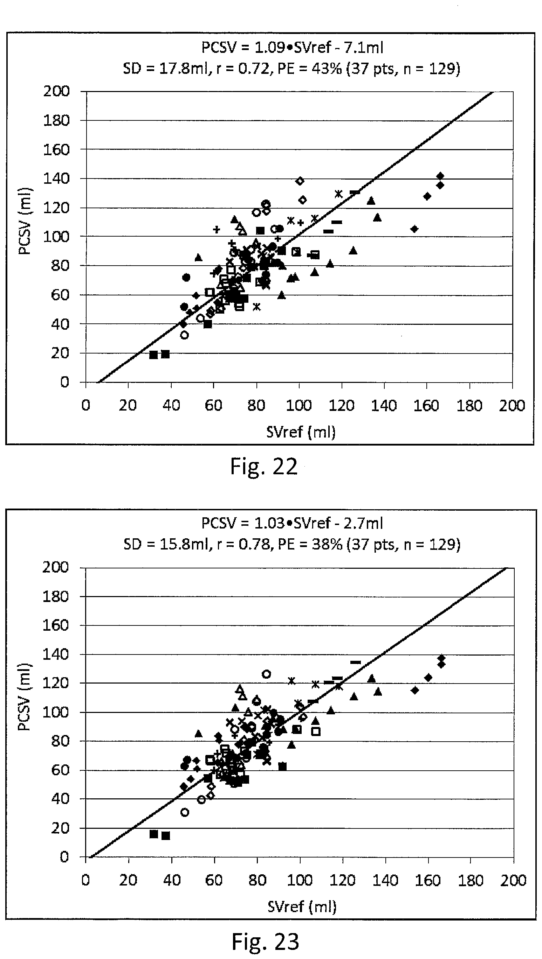

In Figures 22 to 30 different regression diagrams are illustrated which data values are based on measurements and determinations according to conventional and/or inventive method for showing differences in their accuracy.

In Figures 22 to 33 different regression diagrams are given, that show examples of the biologically calibrated noninvasive PCSV calculation versus simultaneously taken corresponding SVref from transpulmonary thermodilution measurements in 37 patients. The PCSV values are based on measurements and determinations according to conventional and/or inventive method for showing differences in their accuracy. Therein SD stands for standard deviation, r for the Pearson correlation coefficient, CR for the concordance rate, PE for percentage error, pts for patients, and n for the number of measurements.

Fig. 22 shows a regression diagram illustrating results of a method for determining a PCSV based on a conventional algorithm (Wesseling algorithm in Chen et al. Comput Cardiol. 2009 Jan 1. The Effect of Signal Quality on Six Cardiac Output Estimators.) for providing the PCSV_uncal. The Wesseling algorithm is an algorithm for providing a PCSV_uncal having no dimension and thus needs to be calibrated to an individual based on the biological characteristic of the individual. The conventional biological calibration of the PCSV_uncal is made based on the BSA as described above. Furthermore, no PCSV adjustment based on a FRP has been applied. The low accuracy and precision of PCSV vs SVref is recognizable in Figure 22, indicated by high SD and PE values and a low correlation coefficient.

Fig. 23 shows a regression diagram illustrating results of a method for determining a PCSV based on an alternative algorithm for providing the PCSV_uncal. Also here the conventional biological calibration parameter BSACal based on BSA and no PCSV adjustment based on FRP were applied. So, in Figure 23 only the algorithm for deriving the PCSV_uncal is different. As explained above with reference to Figure 1b, generally the PCSV_uncal has no dimension and is derived from any an arterial blood pressure waveform, which can be obtained invasively or noninvasively. Based on parameters derived like this a blood pressure curve of an individual is identified and several different parameters are taken into account for forming the PCSV_uncal. In comparison to Figure 22, it can be monitored that the alternative PCSV_uncal algorithm yields higher accuracy and precision than the conventional algorithm. This illustrates that different algorithms can be used for deriving the starting value PCSV _uncal, which is required for the present invention.

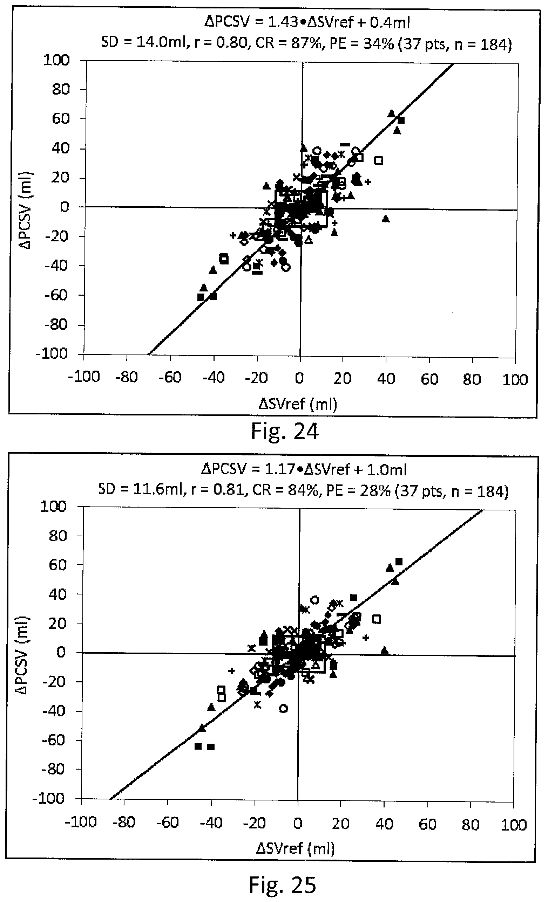

Figures 24 and 25 both are regression diagrams (4-Quadrant-Plots) illustrating changes of PCSV, ΔPCSV, versus the changes of reference thermodilution SVref, ΔSVref, both related to corresponding initial PCSV and SVref values based on the same PCSV_uncal algorithms as in Figure 22 and 23.

Figure 24 shows a regression diagram of ΔPCSV and ΔSVref values based on the conventional algorithm for deriving PCSV_uncal. Further, the BSACal is applied, while FRPs are not considered.

Figure 25 shows a regression diagram of ΔPCSV and ΔSVref values based on the alternative algorithm for deriving PCSV_uncal. Further, the BSACal is applied, while FRPs are not considered. In comparison to Figure 24 applying the alternative algorithm for deriving the PCSV_uncal already improves the accuracy and precision of ΔPCSV vs ΔSVref, indicated by lower SD and PE values and a higher correlation coefficient and CR.

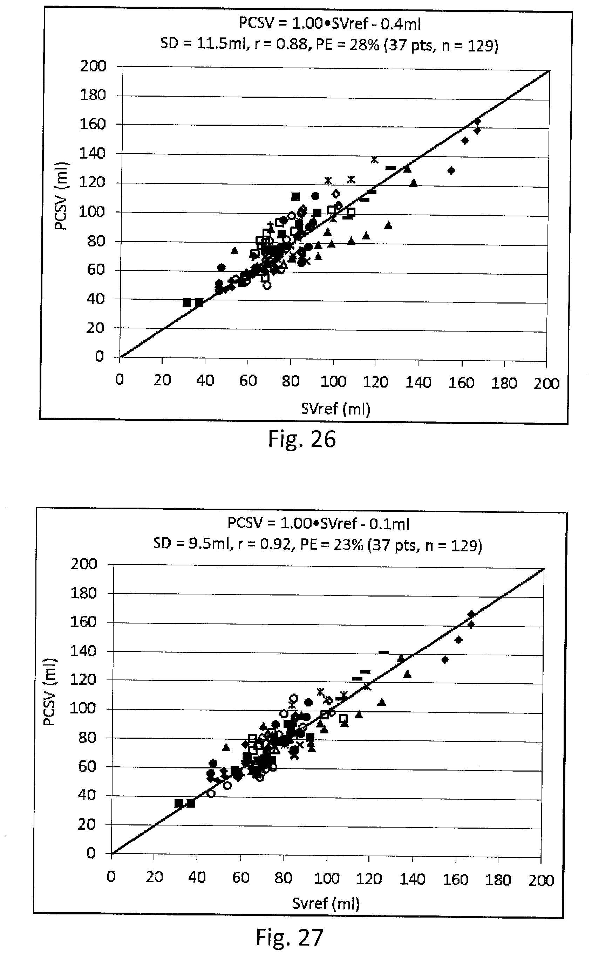

Figure 26 shows a regression diagram illustrating results of a method for determining a PCSV based on the conventional Wesseling algorithm for providing the PCSV_uncal. The biological calibration of the PCSV_uncal is made based on the inventive perfusion parameter, BioCal. Again, no PCSV adjustment based on FRP has been applied. Accuracy and precision of PCSV vs SVref are significantly improved compared to Figure 22, indicated by lower SD and PE values and a higher correlation coefficient. The only change applied from Figure 22 to Figure 26, the application of perfusion parameter BioCal instead of BSACal, improves the determination of PCSV.

Figure 27 shows a regression diagram illustrating results of a method for determining a PCSV based on the alternative algorithm for providing the PCSV_uncal. The biological calibration of the PCSV_uncal is made based on the inventive perfusion parameter, BioCal. Again, no PCSV adjustment based on FRP has been applied. Accuracy and precision of PCSV vs SVref are significantly improved compared to Figure 23 and Figure 26, indicated by lower SD and PE values and a higher correlation coefficient. The only change applied from Figure 23 to Figure 27, the application of perfusion parameter BioCal instead of BSACal, improves the determination of PCSV.

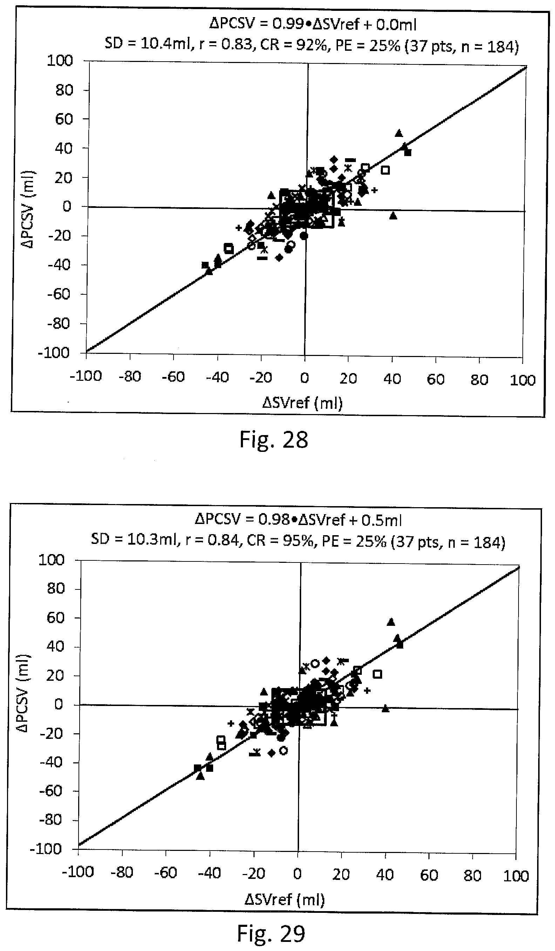

Figures 28 and 29 both are regression diagrams (4-Quadrant-Plots) illustrating changes of PCSV, ΔPCSV, versus the changes of reference thermodilution SVref, ΔSVref, both related to corresponding initial PCSV and SVref values based on the same PCSV_uncal algorithms as in Figure 26 and 27.

Figure 28 shows a regression diagram of ΔPCSV and ΔSVref values based on the conventional algorithm for deriving PCSV_uncal. Further, the inventive perfusion parameter BioCal is applied, while FRPs are not considered. Accuracy and precision of ΔPCSV vs ΔSVref are significantly improved compared to Figure 24, indicated by lower SD and PE values and a higher correlation coefficient and CR. The only change applied from Figure 24 to Figure 28, the application of the inventive perfusion parameter BioCal instead of BSACal, already improves the ability to track true changes of PCSV.

Figure 29 shows a regression diagram of ΔPCSV and ΔSVref values based on the alternative algorithm for deriving PCSV_uncal. Further, the BSACal is applied, while FRPs are not considered. Accuracy and precision of ΔPCSV vs ΔSVref are significantly improved compared to Figure 25, indicated by lower SD and PE values and a higher correlation coefficient and CR. The only change applied from Figure 25 to Figure 29, the application of the inventive perfusion parameter BioCal instead of BSACal, already improves the ability to track true changes of PCSV.

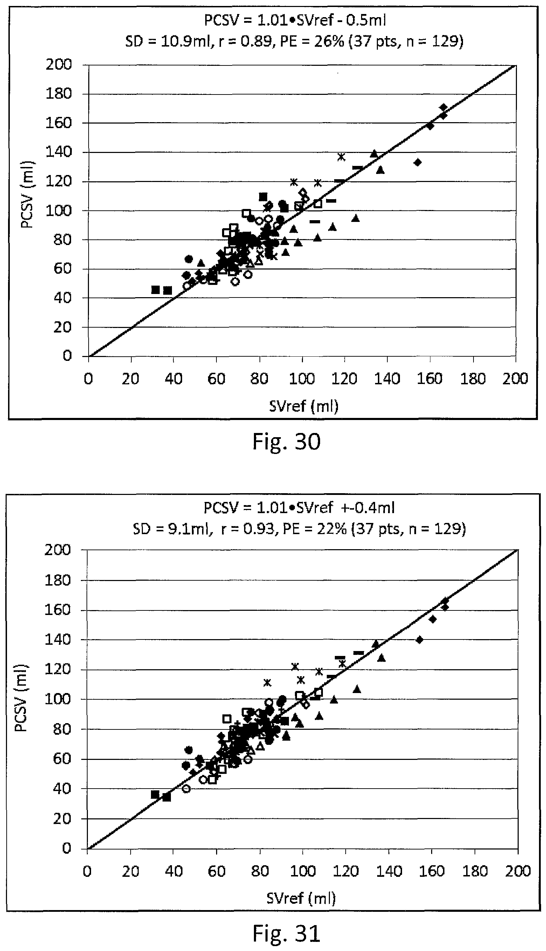

Figure 30 until Figure 33 depict the effect of the FRP function f(FRP_norm) to the sensitivity of the pulse contour algorithm to track true PCSV changes by taking into account FRP norm.

Figure 30 shows a regression diagram illustrating results of a method for determining a PCSV based on the conventional Wesseling algorithm for providing the PCSV_uncal. The biological calibration of the PCSV_uncal is made based on the inventive perfusion parameter, BioCal. Additionally, PCSV adjustment based on f(FRP _norm) to better track true changes has been applied. Accuracy and precision of PCSV vs SVref are slightly improved compared to Figure 26, indicated by lower SD and PE values and a higher correlation coefficient. The only change applied from Figure 26 to Figure 30, the application of f(FRP_norm), further improves the determination of PCSV.

Figure 31 shows a regression diagram illustrating results of a method for determining a PCSV based on the alternative algorithm for providing the PCSV_uncal. The biological calibration of the PCSV_uncal is made based on the inventive perfusion parameter, BioCal. Furthermore, PCSV adjustment based on f(FRP_norm) to better track true changes of PCSV has been applied. Accuracy and precision of PCSV vs SVref are slightly improved compared to Figure 27 and significantly improved compared to Figure 30, indicated by lower SD and PE values and a higher correlation coefficient. The only change applied from Figure 27 to Figure 31, the application of f(FRP_norm), further improves the determination of PCSV and yields the best accuracy and precision in comparison to Figures 22, 23, 26, 27, 30.

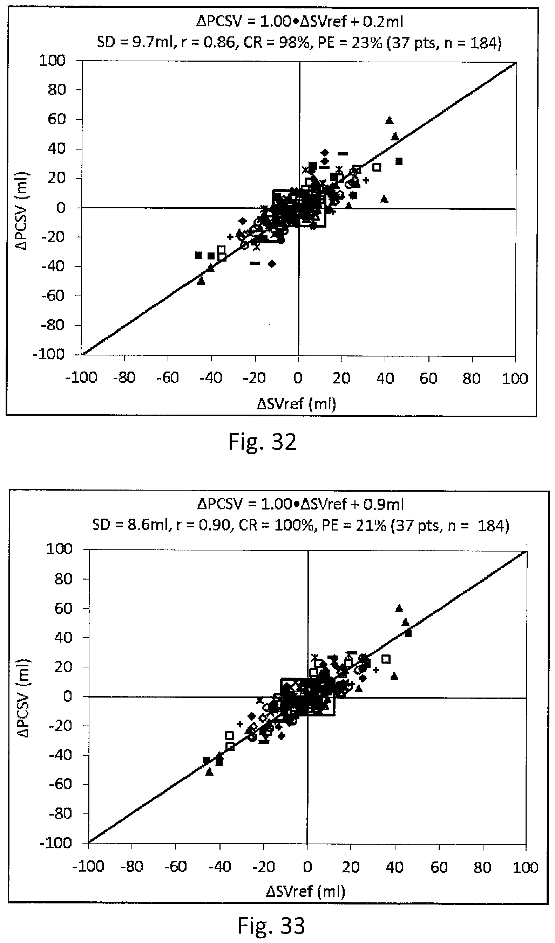

Figures 32 and 33 both are regression diagrams (4-Quadrant-Plots) illustrating changes of PCSV, ΔPCSV, versus the changes of reference thermodilution SVref, ΔSVref, both related to corresponding initial PCSV and SVref values based on the same PCSV_uncal algorithms as in Figure 30 and 31.

Figure 32 shows a regression diagram of ΔPCSV and ΔSVref values based on the conventional algorithm (Wesseling) for deriving PCSV_uncal. Further, the perfusion parameter BioCal is applied. PCSV adjustment based on f(FRP _norm) to better track true changes has been applied. Accuracy and precision of ΔPCSV vs ΔSVref are significantly improved compared to Figure 28, indicated by lower SD and PE values and a higher correlation coefficient and CR. The only change applied from Figure 28 to Figure 32, the application of f(FRP _norm), further improves the ability to track true changes of PCSV.

Figure 33 shows a regression diagram of ΔPCSV and ΔSVref values based on the alternative algorithm for deriving PCSV_uncal. Further, the inventive perfusion parameter BioCal has been applied. PCSV adjustment based on f(FRP _norm) to better track true changes has been applied. Accuracy and precision of ΔPCSV vs ΔSVref are significantly improved compared to Figure 29 and 32, indicated by lower SD and PE values and a higher correlation coefficient and CR. The only change applied from Figure 29 to Figure 33, the application of f(FRP _norm), further improves the ability to track true changes of PCSV and yields the best accuracy and precision in comparison to Figures 24, 25, 28, 29, 32.

So, when comparing the results shown in Figures 31 and 33 with any comparable other regression diagram, it is obvious that the application of the inventive perfusion parameter BioCal and the application of inventive f(FRP_norm) descriptive for the heart-lung interaction of the individual drastically improves the accuracy of the second PCSV_imp being used as a basis for diagnosis and management of the individual.

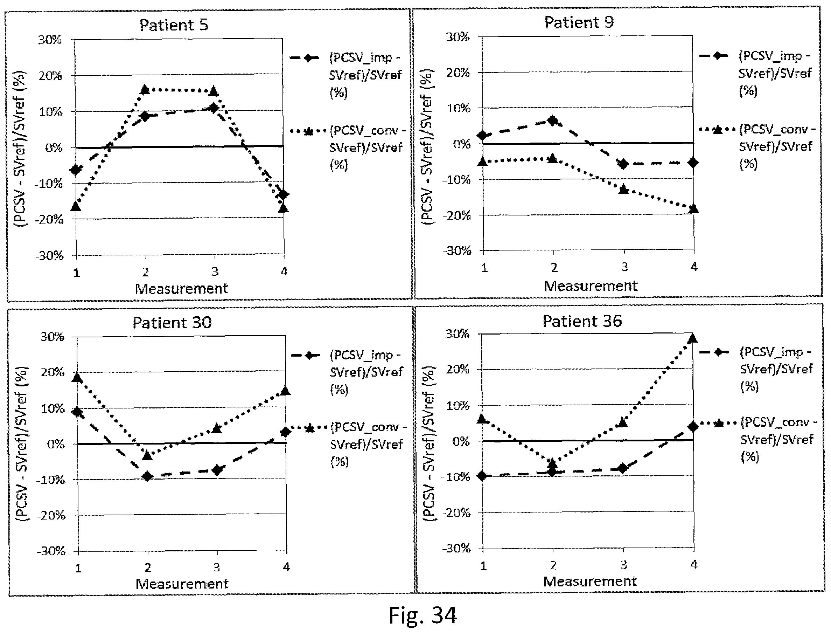

Figure 34 illustrates the relative errors of the second PCSV_imp determined according to the invention and a conventional PCSV _conv, each related to a reference stroke volume SVref. Measurements of four different exemplary patients are illustrated. The relative error for the PCSV_imp determined based on the invention using the alternative algorithm for deriving PCSV_uncal applying BioCal and f(FRP _norm) is calculated as (PCSV_imp-SVref)/SVref, whereas the relative error for the PCSV_conv determined based on the alternative algorithm for deriving PCSV_uncal without applying BioCal and f(FRP _norm) is calculated as (PCSV _conv -SVref)/SVref. As could be easily recognized, the dashed line for the PCSV_imp according to the invention is closer to the 0% line than the dotted line for the PCSV of the state of the art. This applies for all four different patients.

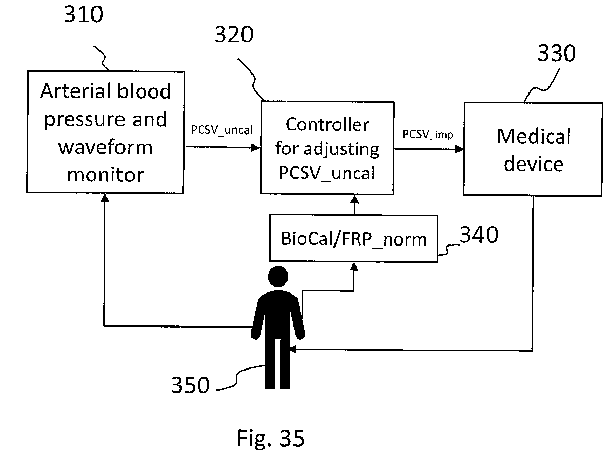

Figure 35 illustrates a schematic apparatus according to an embodiment of the invention. The apparatus substantially comprises a patient monitor 310 performing a function of an arterial blood pressure and waveform measuring and a blood pressure and waveform processing device, i.e. an arterial blood pressure and waveform monitor, a controller 320 for adjusting the derived or processed PCSV_uncal and a medical device 330. Furthermore, there is a parameter unit 340 providing the perfusion parameter BioCal and/or the FRP_norm parameter. This parameter unit 340 might be integrated in the controller 320 or might be realized as a single unit. A patient 350 is connected to the arterial blood pressure and waveform monitor 310. It shall be understood by the skilled in the art person that the illustrated units in figure 35: the controller 320 and the parameter unit 340 might be implemented as functional parts of a single device such as patient monitor 310.

In a simple form a blood pressure cuff or invasive blood pressure measuring apparatus (not illustrated) is connected to the patient 350 to provide a plurality of arterial blood pressures and corresponding waveforms of the patient based on the invasive or non-invasive measured blood pressure.

Based on the measured blood pressure and the resulting waveform a predetermined portion of the pulse pressure wave is extracted by the patient monitor 310, as shown in figure 1b. The blood pressure curve illustrated in figure 1b is extracted from the blood pressure waveform which is measured by use of the blood pressure cuff or invasive blood pressure measuring apparatus which are not illustrated in figure 35. The blood pressure waveform is received by the patient monitor 310 (a corresponding processor of the monitor) and can be processed by filtering, calibration or linearization to extract the blood pressure curve as illustrated in figure 1b. Based on this extracted blood pressure curve an area Asys is determined, which is called systolic pressure area, Asys. The area Asys is enclosed by the blood pressure curve during the systole. This area Asys is used as a value for the first PCSV_uncal which is improved by using the inventive method executed by an inventive functional unit (or apparatus) for adjusting the first PCSV_uncal based on the perfusion parameter and/or based on the fluid responsiveness parameter FRP_norm.

The second PCSV_imp is output by the controller 320 and provided to the medical device 330, which is in its simplest form might be a display (for example, a display ) indicating the second PCSV_imp in ml and/or ml/kg FFM and/or ml/m$^2$ BSA.

In a further advanced embodiment, it is possible to use the second PCSV_imp for controlling an automatic fluid feeder and/or an infusion pump which might be included or coupled to the medical device 330 and which is not illustrated in detail here.

By controlling the e.g. infusion pump based on the second PCSV_imp the patient which might be in an intensive care station could be treated more reliable.

Furthermore, the second PCSV_imp is used by the physician or medical staff for providing a more precise diagnosis and therapy management of the patient.

The parameters for the perfusion, BioCal, or for the fluid responsiveness, FRP_norm are derived based on measurements of characteristics of the patient 350 taken in advance (and stored in a medical database, for example) or during the process of adjusting the first PCSV_uncal which is described in more detail above.

Therefore, the patient monitor can be enabled to determine the stroke volume (SV) of the individual 350 by including a processor arranged to perform the following functions:

(1) provide a first pulse contour stroke volume (PCSV _uncal) based on received one or more characteristics of a measured arterial blood pressure waveform or providing a conventionally derived pulse contour stroke volume (PCSV_conv).

This can achieved by receiving the pressure waveforms from the blood pressure cuff or invasive blood pressure measuring apparatus and performing analyses described above.

(2) determine at least one perfusion parameter (BioCal) descriptive for the perfusion through the fat free mass and the adipose mass of a body of the individual, and/or

- determining at least one fluid responsiveness parameter function f (FRP_norm) depending on a fluid responsiveness parameter (FRP_norm) descriptive for a heart-lung interaction of the individual;

At this stage the appropriate patient medical data required for this analyses are either retrieved from the medical data base by the patient monitor (its processor via the parameter unit 340, for example); and/ or manually input by the physician during the process of adjusting the first PCSV_uncal.

(3) adjust the first pulse contour stroke volume (PCSV _uncal) based on at the least one of the perfusion parameter (BioCal) and/or the fluid responsiveness parameter function f(FRP_norm), or

- adjust the conventionally derived pulse contour stroke volume (PCSV_conv) based on the fluid responsiveness parameter function f (FRP_norm) to provide a second pulse contour stroke volume (PCSV_imp).

This second pulse contour stroke volume (PCSV _imp) can be displayed on a display of the patient monitor; the PCSV_imp can be further input to other medical devices arranged to provide/ control a therapy to the patient.

The processor of the patient monitor can be arranged to perform any of the steps of the described above flow charts, which are illustrated in figures 1, 2, 8, 9, 10 and 14.

## Claims

1. A computer implemented method for determining a stroke volume (SV) of an individual, comprising the steps of:

   - a) providing (S100) a first pulse contour stroke volume (PCSV _uncal) based on one or more characteristics of a measured arterial blood pressure waveform,

     determining (S200) at least one perfusion parameter (BioCal) descriptive for the perfusion through the fat free mass and the adipose mass of a body of the individual, and/or determining (S300) at least one fluid responsiveness parameter function f (FRP_norm) depending on a fluid responsiveness parameter (FRP _norm) descriptive for a heart-lung interaction of the individual, and adjusting (S280, S350) the first pulse contour stroke volume (PCSV_uncal) based on at the least one of the perfusion parameter (BioCal) and/or the fluid responsiveness parameter function f (FRP_norm) to provide a second pulse contour stroke volume (PCSV_imp), or

   - b) providing a conventionally derived pulse contour stroke volume (PCSV_conv),

     determining at least one fluid responsiveness parameter function f (FRP _norm) depending on a fluid responsiveness parameter (FRP _norm) descriptive for a heart-lung interaction of the individual, and adjusting the conventionally derived pulse contour stroke volume (PCSV _conv) based on the fluid responsiveness parameter function f (FRP_norm) to provide a second pulse contour stroke volume (PCSV _imp).

2. The method as claimed in claim 1, wherein the provided first pulse contour stroke volume (PCSV _uncal) and/or the provided conventionally derived pulse contour stroke volume (PCSV_conv) are based on non-invasive measurement or invasive measurement of arterial blood pressure waveform.

3. The method as claimed in one of the claims 1 or 2, wherein the step of determining at least one fluid responsiveness parameter (FRP _norm) is based on at least one of a pulse pressure variation (PPV), a mean left ventricular ejection pressure variation MEPV, a stroke volume variation (SVV), a systolic pressure variation (SPV), a left ventricular systolic pressure area variation (SPAV), a photoplethysmographic variability index (PVI), or any other fluid responsiveness parameter with appropriate sensitivity and specificity, or a combination thereof.

4. The method as claimed in any one of the preceding claims, wherein the fluid responsiveness parameter (FRP _norm) is determined by applying a fluid responsiveness normalization function (f_FRP_MV) for mechanical ventilation or the fluid responsiveness parameter (FRP _norm) is determined by applying a fluid responsiveness normalization function (f_FRP_SB) for spontaneous breathing.

5. The method as claimed in any one of the preceding claims, wherein the fluid responsiveness parameter (FRP _norm) is determined by considering a compliance of a respiratory system (Crs) of the individual, wherein the compliance of the respiratory system (Crs) comprises lung compliance (Cls) and chest wall compliance (Ccw).

6. The method as claimed in any one of the preceding claims, wherein the fluid responsiveness parameter (FRP_norm) is normalized by using a FRP normalization parameter (TVnorm), wherein the FRP normalization parameter (TVnorm) is normalized to the fat free body mass (FFM), predicted body weight (PBW) or to the body weight (W).

7. The method as claimed in any one of the preceding claims 5 or 6, wherein the fluid responsiveness parameter (FRP _norm) is adjusted to mechanical ventilation (MV) by use of predetermined semi-quantitative information about the chest wall compliance (Ccw).

8. The method as claimed in any one of the preceding claims 5 or 6, wherein the fluid responsiveness parameter (FRP _norm) is normalized for spontaneous breathing (SB) by use of the lung compliance (Cls) and respiration rate (RR).

9. The method as claimed in any one of the preceding claims, wherein the step of determining a perfusion parameter (BioCal) descriptive for the perfusion of the individual through the fat free mass and the adipose mass comprises:

   - determining a fat free mass related pulse contour stroke volume calibration factor (PCSV_FFM_Cal), and/or
   - determining an adipose mass related pulse contour stroke volume calibration factor (PCSV_AM_Cal).

10. The method according to claim 9, further comprising determining an individual demographic parameter comprising:

    - determining the fat fee mass (FFM),
    - determining the adipose mass (AM),
    - a height (h) of the individual,
    - a biological gender (male/female/transgender) of the individual, and
    - an age (y) of the individual.

11. The method according to any one of claims 9 or 10, wherein the fat free mass related pulse contour stroke volume calibration factor (PCSV_FFM_Cal) is determined by using a perfusion coefficient B_FFM relating to the fat free mass perfusion and/or the adipose mass pulse contour stroke volume calibration factor (PCSV _AM Cal) is determined by using a perfusion coefficient B_AM relating to the adipose mass perfusion.

12. The method according to any one of claims 9, 10 or 11, wherein for determining the fat free mass related pulse contour stroke volume calibration factor (PCSV_FFM_Cal) and/or the adipose mass pulse contour stroke volume calibration factor (PCSV_AM_Cal) the fat free mass layer thickness (FFM/H) and the adipose mass layer thickness (AM/H) are considered.

13. An apparatus for determining a stroke volume (SV) of an individual, comprising:

    - a) an arterial blood pressure and waveform monitor (310) for providing a first pulse contour stroke volume (PCSV_uncal) based on medical data obtained from an individual (350) connected to the arterial blood pressure and waveform monitor (310),

      a parameter unit (340) for providing a perfusion parameter (BioCal) and/or a heart-lung interaction descriptive parameter (FRP _norm) of the individual (350),
      a controller (320) for adjusting the first pulse contour stroke volume (PCSV_uncal) based on the perfusion parameter (BioCal) and/or based on the heart-lung interaction descriptive parameter (FRP _norm) to provide a second pulse contour stroke volume (PCSV_imp), or

    - b) an arterial blood pressure and waveform monitor (310) for providing a conventional pulse contour stroke volume (PCSV_conv) based on medical data obtained from an individual (350) connected to the arterial blood pressure and waveform monitor (310),

a parameter unit (340) for providing a heart-lung interaction descriptive parameter (FRP _norm) of the individual (350),
a controller (320) for adjusting the conventional pulse contour stroke volume (PCSV _conv) based on the heart-lung interaction descriptive parameter (FRP_norm) to provide a second pulse contour stroke volume (PCSV _imp).

**14.** The apparatus of claim 13 further comprising:

- a medical device (330) arranged to receive the second pulse contour stroke volume (PCSV _imp) and output the second pulse contour stroke volume (PCSV _imp) or related data therefrom.

**Patentansprüche**

**1.** Computergestütztes Verfahren zum Bestimmen eines Schlagvolumens (SV) einer Person, das die folgenden Schritte umfasst:

- a) Bereitstellen (S100) eines ersten Pulskontur-Schlagvolumens (PCSV_uncal) basierend auf einer oder mehreren Eigenschaften einer gemessenen arteriellen Blutdruckwellenform,

Bestimmen (S200) mindestens eines Perfusionsparameters (BioCal), der die Perfusion durch die fettfreie Masse und die Fettmasse eines Körpers der Person beschreibt, und/oder Bestimmen (S300) mindestens einer Fluidreaktivitätsparameterfunktion f (FRP_norm) in Abhängigkeit von einem Fluidreaktivitätsparameter (FRP_norm), der eine Herz-Lungen-Interaktion der Person beschreibt, und Einstellen (S280, S350) des ersten Pulskontur-Schlagvolumens (PCSV_uncal) basierend auf mindestens einem des Perfusionsparameters (BioCal) und/oder der Fluidreaktivitätsparameterfunktion f (FRP_norm) zum Bereitstellen eines zweiten Pulskontur-Schlagvolumens (PCSV_imp), oder

- b) Bereitstellen eines konventionell abgeleiteten Pulskontur-Schlagvolumens (PCSV_conv),

Bestimmen mindestens einer Fluidreaktivitätsparameterfunktion f (FRP_norm) in Abhängigkeit von einem Fluidreaktivitätsparameter (FRP_norm), der eine Herz-Lungen-Interaktion der Person beschreibt, und Einstellen des konventionell abgeleiteten Pulskontur-Schlagvolumens (PCSV_conv) basierend auf der Fluidreaktivitätsparameterfunktion f (FRP_norm) zum Bereitstellen eines zweiten Pulskontur-Schlagvolumens (PCSV_imp).

**2.** Verfahren nach Anspruch 1, wobei das bereitgestellte erste Pulskontur-Schlagvolumen (PCSV_uncal) und/oder das bereitgestellte konventionell abgeleitete Pulskontur-Schlagvolumen (PCSV_conv) auf nicht-invasiver Messung oder invasiver Messung der arteriellen Blutdruckwellenform basieren.

**3.** Verfahren nach einem der Ansprüche 1 oder 2, wobei der Schritt des Bestimmens mindestens eines Fluidreaktivitätsparameters (FRP_norm) auf mindestens einer von einer Pulsdruckvariation (PPV), einer mittleren linksventrikulären Auswurfdruckvariation MEPV, einer Schlagvolumenvariation (SVV), einer systolischen Druckvariation (SPV), einer linksventrikulären systolischen Druckflächenvariation (SPAV), einem photoplethysmographischen Variabilitätsindex (PVI) oder einem beliebigen anderen Fluidreaktivitätsparameter mit geeigneter Sensitivität und Spezifität oder einer Kombination davon basiert.

**4.** Verfahren nach einem der vorstehenden Ansprüche, wobei der Fluidreaktivitätsparameter (FRP_norm) durch Anwenden einer Fluidreaktivitäts-Normalisierungsfunktion (f_FRP_MV) für die mechanische Beatmung bestimmt wird, oder der Fluidreaktivitätsparameter (FRP_norm) durch Anwenden einer Fluidreaktivität-Normalisierungsfunktion (f_FRP_SB) für die Spontanatmung bestimmt wird.

**5.** Verfahren nach einem der vorstehenden Ansprüche, wobei der Fluidreaktivitätsparameter (FRP_norm) unter Berücksichtigung einer Nachgiebigkeit eines Atmungssystems (Crs) der Person bestimmt wird, wobei die Nachgiebigkeit des Atmungssystems (Crs) die Lungennachgiebigkeit (Cls) und die Brustwandnachgiebigkeit (Ccw) umfasst.

6. Verfahren nach einem der vorstehenden Ansprüche, wobei der Fluidreaktivitätsparameter (FRP_norm) durch Verwendung eines FRP-Normalisierungsparameters (TVnorm) normalisiert wird, wobei der FRP-Normalisierungsparameter (TVnorm) auf die fettfreie Körpermasse (FFM), das vorhergesagte Körpergewicht (PBW) oder auf das Körpergewicht (W) normalisiert wird.

7. Verfahren nach einem der vorstehenden Ansprüche 5 oder 6, wobei der Fluidreaktivitätsparameter (FRP_norm) durch Verwendung vorbestimmter halbquantitativer Informationen über die Brustkorbnachgiebigkeit (Ccw) an die mechanische Beatmung (MV) eingestellt wird.

8. Verfahren nach einem der vorstehenden Ansprüche 5 oder 6, wobei der Fluidreaktivitätsparameter (FRP_norm) für die Spontanatmung (SB) durch Verwendung der Lungennachgiebigkeit (Cls) und der Atemfrequenz (RR) normalisiert wird.

9. Verfahren nach einem der vorstehenden Ansprüche, wobei der Schritt des Bestimmens eines Perfusionsparameters (BioCal), der die Perfusion der Person durch die fettfreie Masse und die Fettmasse beschreibt, umfasst:

- Bestimmen eines die fettfreie Masse betreffenden Kalibrierfaktors für das Pulskontur-Schlagvolumen (PCSV_FFM_Cal), und/oder
- Bestimmen eines die Fettmasse betreffenden Kalibrierfaktors für das Pulskontur-Schlagvolumen (PCSV_AM_Cal).

10. Verfahren nach Anspruch 9, das weiter das Bestimmen eines individuellen demographischen Parameters umfasst, umfassend:

- Bestimmen der fettfreien Masse (FFM),
- Bestimmen der Fettmasse (AM),
- eine Größe (h) der Person,
- ein biologisches Geschlecht (männlich/weiblich/transgeschlechtlich) der Person und
- ein Alter (y) der Person.

11. Verfahren nach einem der Ansprüche 9 oder 10, wobei der die fettfreie Masse betreffende Kalibrierfaktor für das Pulskontur-Schlagvolumen (PCSV_FFM_Cal) durch Verwendung eines Perfusionskoeffizienten B_FFM in Bezug auf die Perfusion der fettfreien Masse bestimmt wird, und/oder der die Fettmasse betreffende Kalibrierfaktor für das Pulskontur-Schlagvolumen (PCSV_AM_Cal) durch Verwendung eines Perfusionskoeffizienten B_AM in Bezug auf die Perfusion der Fettmasse bestimmt wird.

12. Verfahren nach einem der Ansprüche 9, 10 oder 11, wobei zur Bestimmung des die fettfreie Masse betreffenden Kalibrierfaktors für das Pulskontur-Schlagvolumen (PCSV_FFM_Cal) und/oder des die Fettmasse betreffenden Kalibrierungsfaktors für das Pulskontur-Schlagvolumen (PCSV_AM_Cal) die Schichtdicke der fettfreien Masse (FFM/H) und die Schichtdicke der Fettmasse (AM/H) berücksichtigt werden.

13. Einrichtung zum Bestimmen eines Schlagvolumens (SV) einer Person, umfassend:

- a) einen Monitor für arteriellen Blutdruck und Wellenform (310) zum Bereitstellen eines ersten Pulskontur-Schlagvolumens (PCSV_uncal) basierend auf medizinischen Daten, die von einer Person (350) erhalten werden, die an den Monitor für arteriellen Blutdruck und Wellenform (310) angeschlossen ist,

eine Parametereinheit (340) zum Bereitstellen eines Perfusionsparameters (BioCal) und/oder eines die Herz-Lungen-Interaktion beschreibenden Parameters (FRP_norm) der Person (350), eine Steuereinheit (320) zum Einstellen des ersten Pulskontur-Schlagvolumens (PCSV_uncal) basierend auf dem Perfusionsparameter (BioCal) und/oder basierend auf dem die Herz-Lungen-Interaktion beschreibenden Parameter (FRP_norm) zum Bereitstellen eines zweiten Pulskontur-Schlagvolumens (PCSV_imp), oder

- b) einen Monitor für arteriellen Blutdruck und Wellenform (310) zum Bereitstellen eines konventionellen Pulskontur-Schlagvolumens (PCSV_conv) basierend auf medizinischen Daten, die von einer Person (350) erhalten werden, die an den Monitor für arteriellen Blutdruck und Wellenform (310) angeschlossen ist,

eine Parametereinheit (340) zum Bereitstellen eines die Herz-Lungen-Interaktion beschreibenden Parameters (FRP_norm) der Person (350),
eine Steuereinheit (320) zum Einstellen des konventionellen Pulskontur-Schlagvolumens (PCSV_conv) basierend auf dem die Herz-Lungen-Interaktion beschreibenden Parameter (FRP_norm) zum Bereitstellen eines zweiten Pulskontur-Schlagvolumens (PCSV_imp).

**14.** Einrichtung nach Anspruch 13, weiter umfassend:

- eine medizinische Vorrichtung (330), die dazu angeordnet ist, das zweite Pulskontur-Schlagvolumen (PCSV_imp) zu empfangen und das zweite Pulskontur-Schlagvolumen (PCSV_imp) oder damit verbundene Daten auszugeben.

**Revendications**

**1.** Procédé mis en oeuvre par ordinateur pour déterminer un volume systolique (SV) d'un individu, comprenant les étapes consistant à :

- a) fournir (S100) un premier volume systolique à contour d'impulsion (PCSV_uncal) sur la base d'une ou de plusieurs caractéristiques d'une forme d'onde de pression artérielle mesurée,

déterminer (S200) au moins un paramètre de perfusion (BioCal) descriptif de la perfusion à travers la masse maigre et la masse adipeuse d'un corps de l'individu et/ou déterminer (S300) au moins une fonction f de paramètre de réactivité aux fluides (FRP_norm) en fonction d'un paramètre de réactivité aux fluides (FRP_norm) descriptif d'une interaction coeur-poumons de l'individu, et
ajuster (S280, S350) le premier volume systolique à contour d'impulsion (PCSV_uncal) sur la base d'au moins un du paramètre de perfusion (BioCal) et/ou de la fonction f de paramètre de réactivité aux fluides (FRP_norm) pour fournir un second volume systolique à contour d'impulsion (PCSV_imp), ou

- b) fournir un volume systolique à contour d'impulsion dérivé de manière conventionnelle (PCSV_conv),

déterminer au moins une fonction f de paramètre de réactivité aux fluides (FRP_norm) en fonction d'un paramètre de réactivité aux fluides (FRP_norm) descriptif d'une interaction coeur-poumons de l'individu, et
ajuster le volume systolique à contour d'impulsion dérivé de manière conventionnelle (PCSV_conv) sur la base de fonction f de paramètre de réactivité au fluide (FRP_norm) pour fournir un second volume systolique à contour d'impulsion (PCSV_imp).

**2.** Procédé selon la revendication 1, dans lequel le premier volume systolique à contour d'impulsion fourni (PCSV_uncal) et/ou le volume systolique à contour d'impulsion dérivé de manière conventionnelle fourni (PCSV_conv) sont basés sur une mesure non invasive ou une mesure invasive de la forme d'onde de pression artérielle.

**3.** Procédé selon l'une des revendications 1 ou 2, dans lequel l'étape de détermination d'au moins un paramètre de réactivité aux fluides (FRP_norm) est basée sur au moins l'un d'une variation de pression d'impulsion (PPV), d'une variation de pression d'éjection ventriculaire gauche moyenne MEPV, d'une variation de volume systolique (SVV), d'une variation de pression systolique (SPV), d'une variation d'aire de pression systolique ventriculaire gauche (SPAV), d'un indice de variabilité photopléthysmographique (PVI), ou de tout autre paramètre de réactivité aux fluides avec une sensibilité et une spécificité appropriées, ou d'une combinaison de ceux-ci.

**4.** Procédé selon l'une quelconque des revendications précédentes, dans lequel le paramètre de réactivité aux fluides (FRP_norm) est déterminé en appliquant une fonction de normalisation de réactivité aux fluides (f_FRP_MV) pour une ventilation mécanique ou
le paramètre de réactivité aux fluides (FRP_norm) est déterminé en appliquant une fonction de normalisation de réactivité aux fluides (f_FRP_SB) pour une respiration spontanée.

**5.** Procédé selon l'une quelconque des revendications précédentes, dans lequel le paramètre de réactivité aux fluides (FRP_norm) est déterminé en considérant une compliance d'un système respiratoire (Crs) de l'individu, dans lequel la compliance du système respiratoire (Crs) comprend la compliance pulmonaire (Cls) et la compliance de paroi thoracique (Ccw).

6. Procédé selon l'une quelconque des revendications précédentes, dans lequel le paramètre de réactivité aux fluides (FRP_norm) est normalisé en utilisant un paramètre de normalisation de FRP (TVnorm), dans lequel le paramètre de normalisation de FRP (TVnorm) est normalisé par rapport à la masse corporelle maigre (FFM), au poids corporel prédit (PBW) ou au poids corporel (W).

7. Procédé selon l'une quelconque des revendications précédentes 5 ou 6, dans lequel le paramètre de réactivité aux fluides (FRP_norm) est ajusté à une ventilation mécanique (VM) par l'utilisation d'informations semi-quantitatives prédéterminées concernant la compliance de paroi thoracique (Ccw).

8. Procédé selon l'une quelconque des revendications précédentes 5 ou 6, dans lequel le paramètre de réactivité aux fluides (FRP_norm) est normalisé pour une respiration spontanée (SB) par l'utilisation de la compliance pulmonaire (Cls) et de la fréquence respiratoire (RR).

9. Procédé selon l'une quelconque des revendications précédentes, dans lequel l'étape de détermination d'un paramètre de perfusion (BioCal) descriptif de la perfusion de l'individu à travers la masse maigre et la masse adipeuse comprend :

- la détermination d'un facteur d'étalonnage de volume systolique à contour d'impulsion associé à la masse maigre (PCSV_FFM_Cal), et/ou
- la détermination d'un facteur d'étalonnage de volume systolique à contour d'impulsion associé à la masse adipeuse (PCSV_AM_Cal).

10. Procédé selon la revendication 9, comprenant en outre la détermination d'un paramètre démographique individuel comprenant :

- la détermination de la masse maigre (FFM),
- la détermination de la masse adipeuse (MA),
- la taille (h) de l'individu,
- le genre biologique (masculin/féminin/transgenre) de l'individu, et
- l'âge (y) de l'individu.

11. Procédé selon l'une quelconque des revendications 9 ou 10, dans lequel le facteur d'étalonnage de volume systolique à contour d'impulsion associé à la masse maigre (PCSV_FFM_Cal) est déterminé en utilisant un coefficient de perfusion B_FFM se rapportant à la perfusion de masse maigre et/ou le facteur d'étalonnage de volume systolique à contour d'impulsion de masse adipeuse (PCSV_AM_Cal) est déterminé en utilisant un coefficient de perfusion B_AM se rapportant à la perfusion de masse adipeuse.

12. Procédé selon l'une quelconque des revendications 9, 10 ou 11, dans lequel pour déterminer le facteur d'étalonnage de volume systolique à contour d'impulsion associé à la masse maigre (PCSV_FFM_Cal) et/ou le facteur d'étalonnage de volume systolique à contour d'impulsion de masse adipeuse (PCSV_AM_Cal), l'épaisseur de couche de masse maigre (FFM/H) et l'épaisseur de couche de masse adipeuse (AM/H) sont considérées.

13. Appareil pour déterminer un volume systolique (SV) d'un individu, comprenant :

- a) un moniteur de pression artérielle et de forme d'onde (310) pour fournir un premier volume systolique à contour d'impulsion (PCSV_uncal) sur la base de données médicales obtenues auprès d'un individu (350) connecté au moniteur de pression artérielle et de forme d'onde (310),

une unité de paramètre (340) pour fournir un paramètre de perfusion (BioCal) et/ou un paramètre descriptif d'interaction coeur-poumons (FRP_norm) de l'individu (350),
un dispositif de commande (320) pour ajuster le premier volume systolique à contour d'impulsion (PCSV_uncal) sur la base du paramètre de perfusion (BioCal) et/ou sur la base du paramètre descriptif d'interaction coeur-poumons (FRP_norm) pour fournir un second volume systolique à contour d'impulsion (PCSV_imp), ou

- b) un moniteur de pression artérielle et de forme d'onde (310) pour fournir un volume systolique à contour d'impulsion conventionnel (PCSV_conv) sur la base de données médicales obtenues auprès d'un individu (350) connecté au moniteur de pression artérielle et de forme d'onde (310),

une unité de paramètre (340) pour fournir un paramètre descriptif d'interaction coeur-poumons (FRP_norm) de l'individu (350),
un dispositif de commande (320) pour ajuster le volume systolique à contour d'impulsion conventionnel (PCSV_conv) sur la base du paramètre descriptif d'interaction coeur-poumons (FRP_norm) pour fournir un second volume systolique à contour d'impulsion (PCSV_imp).

14. Appareil selon la revendication 13, comprenant en outre :

- un dispositif médical (330) agencé pour recevoir le second volume systolique à contour d'impulsion (PCSV_imp) et fournir en sortie le volume systolique à contour d'impulsion (PCSV_imp) ou des données associées à partir de celui-ci.

```
┌─────────────────────────────────┐
│   Providing a first PCSV_uncal  │ ⌇ S100
└─────────────────────────────────┘
                 │
                 ▼
┌─────────────────────────────────┐
│      Determining perfusion       │
│       parameter BioCal           │ ⌇ S200
└─────────────────────────────────┘
                 │
                 ▼
┌─────────────────────────────────┐
│   Adjusting the first PCSV_uncal │
│        based on the perfusion    │ ⌇ S280
│        parameter BioCal          │
└─────────────────────────────────┘
                 │
                 ▼
┌─────────────────────────────────┐
│    Outputting second PCSV_imp    │ ⌇ S290
└─────────────────────────────────┘
```

Fig. 1

```
┌─────────────────────────────────────┐
│ Determining one or more characteristics │
│  of an arterial blood pressure waveform │
└─────────────────────────────────────┘  ⌇ S70
                 │
                 ▼
┌─────────────────────────────────┐
│      Identifying a portion of the │
│      arterial pressure waveform   │  ⌇ S80
└─────────────────────────────────┘
                 │
                 ▼
┌─────────────────────────────────┐
│   Determining a first PCSV_uncal │
│    based on at least one portion of │ ⌇ S85
│    the arterial pressure waveform │
└─────────────────────────────────┘
                 │
                 ▼
┌─────────────────────────────────┐
│   Outputting a first PCSV_uncal  │ ⌇ S90
└─────────────────────────────────┘
```

Fig. 1a

Providing a first PCSV_uncal

Fig. 1b

Fig. 2

Fig. 3

Fig. 4

Fig. 5

Fig. 6

| Input: Degree of Muscle Tone | | |
| --- | --- | --- |
| Output: Effects of FFM and AM layer thickness on B_FFM, AM_B_FFM_Red | | |
| normal | reduced | absent |
| ≈ 0 | ↑ | ↑↑ |

Fig. 7

Providing a first PCSV_uncal — S100

Determining fluid responsiveness parameter function f(FRP_norm) — S300

Adjusting the first PCSV_uncal based on the fluid responsiveness parameter function f(FRP_norm) — S350

Outputting second PCSV_imp — S390

Fig. 8

Determining at least one FRP out of pulse pressure
variation (PPV), a mean left ventricular ejection
pressure variation (MEPV), a stroke volume
variation (SVV), a systolic pressure variation (SPV),
a systolic pressure area variation (SPAV), a
photoplethysmographic variability index (PVI), or
any FRP with appropriate sensitivity and specifity

S302

S304
Outputting a
single FRP (%)

at least two
FRP determined
No

S303

Yes

Select one FRP as guiding FRP

S305

S306

Adjust at least one of the other FRP to a
comparable level and/or range of the guiding FRP
by applying regression analysis

S307

Generating a combined FRP (%) based on the
guiding FRP and at least one of the adjusted other
FRP

Outputting the combined FRP (%)

S308

Fig. 9

## Mechanical ventilation

| Providing a normalized tidal volume, TVnorm, normalized to fat free mass FFM | S310 |
| Determining a FRP normalization function, f_FRP_MV, based on the normalized tidal volume TVnorm | S312 |
| Adjust f_FRP_MV to different conditions of the compliance of the chest wall, Ccw | S314 |
| Apply f_FRP_MV on single/combined FRP to receive FRP_norm | S316 |
| Apply adjustment coefficients, a, b, c, d to FRP_norm to receive f(FRPnorm) | S318 |
| Outputting a normalized fluid responsiveness parameter function f(FRP_norm) | S320 |

Fig. 10

Fig. 11

Lungs
Chest wall
(rib cage and
diaphragm)

HLI

Intrathoracic low blood
pressure capacitance system

| MV, Ccw ↑ | MV, Ccw − | MV, Ccw ↓↓↓ |
|:---:|:---:|:---:|
| | | |

Fig. 12

Fig. 13

| Input: Degree of Ccw | | | | | |
|---|---|---|---|---|---|
| Output: Degree of f_FRP_MV adjustment to be applied | | | | | |
| Ccw | ↑ | − | ↓ | ↓↓ | ↓↓↓ |
| f_FRP_MV adjustment | ↑ | − | ↓ | ↓↓ | ↓↓↓ |

## Spontaneous breathing

tidal volume TV available — S410

No

S420

Yes

S430

Determining a FRP normalization function, f_FRP_SB_RR, based on an approximation function f_FRP(RR)

Determining a FRP normalization function, f_FRP_SB_TV, based on the tidal volume TV

S440

Adjust respiration rate normalization function f_FRP_SB to different conditions of the compliance of the lung, Cls

S450

Apply f_FRP_SB on FRP to receive FRP_norm

S460

Apply adjustment coefficients, a, b, c, d to FRP_norm to receive f(FRPnorm)

S470

Outputting a normalized fluid responsiveness parameter function f(FRP_norm)

Fig. 14

Fig. 15

Fig. 16

Fig. 17

| Input: Level of TV | | | | | |
|---|---|---|---|---|---|
| Output: Degree of f_FRP_SB adjustment to be applied | | | | | |
| TV | ↓ | – | ↑ | ↑↑ | ↑↑↑ |
| f_FRP_SB adjustment | ↑ | – | ↓ | ↓↓ | ↓↓↓ |

| SB, Cls – | SB, Cls ↓ | SB, Cls ↓↓↓ |
|---|---|---|

Fig. 18

| Input: Degree of Cls | | | |
|---|---|---|---|
| Output: Degree of f_FRP_SB adjustment to be applied | | | |
| Cls | – | ↓ | ↓↓ | ↓↓↓ |
| f_FRP_SB adjustment | – | ↓ | ↓↓ | ↓↓↓ |

Fig. 19

Fig. 20

Fig. 21

Fig. 22

Fig. 23

ΔPCSV = 1.43•ΔSVref + 0.4ml
SD = 14.0ml, r = 0.80, CR = 87%, PE = 34% (37 pts, n = 184)

Fig. 24

ΔPCSV = 1.17•ΔSVref + 1.0ml
SD = 11.6ml, r = 0.81, CR = 84%, PE = 28% (37 pts, n = 184)

Fig. 25

**PCSV = 1.00•SVref - 0.4ml**
**SD = 11.5ml, r = 0.88, PE = 28% (37 pts, n = 129)**

Fig. 26

**PCSV = 1.00•SVref - 0.1ml**
**SD = 9.5ml, r = 0.92, PE = 23% (37 pts, n = 129)**

Fig. 27

$$\Delta PCSV = 0.99 \cdot \Delta SVref + 0.0ml$$
SD = 10.4ml, r = 0.83, CR = 92%, PE = 25% (37 pts, n = 184)

Fig. 28

$$\Delta PCSV = 0.98 \cdot \Delta SVref + 0.5ml$$
SD = 10.3ml, r = 0.84, CR = 95%, PE = 25% (37 pts, n = 184)

Fig. 29

PCSV = 1.01•SVref - 0.5ml
SD = 10.9ml, r = 0.89, PE = 26% (37 pts, n = 129)

Fig. 30

PCSV = 1.01•SVref +-0.4ml
SD = 9.1ml, r = 0.93, PE = 22% (37 pts, n = 129)

Fig. 31

Fig. 32

Fig. 33

Fig. 34

310

320

330

Arterial blood pressure and waveform monitor

PCSV_uncal →

Controller for adjusting PCSV_uncal

PCSV_imp →

Medical device

BioCal/FRP_norm  340

350

Fig. 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140073962 A1
- US 20130006126 A1
- EP 2502555 A1

- US 20150201873 A1
- WO 2017100188 A2
- WO 2009014420 A1

**Non-patent literature cited in the description**

- **CHEN et al.** *Comput Cardiol.*, 01 January 2009
- **YU et al.** *BMC Pharmacol Toxicol.*, 14 October 2013

- **COLLIS et al.** *Circulation*, 13 February 2001, vol. 103 (6)
- **CORDEN et al.** *J Cardiovasc Magn Reson.*, 31 May 2016, vol. 18 (1), 32